(19) Europäisches Patentamt
European Patent Office
Office européen des brevets

(11) **EP 4 248 954 A1**

(12) **EUROPEAN PATENT APPLICATION**
published in accordance with Art. 153(4) EPC

(43) Date of publication:
**27.09.2023 Bulletin 2023/39**

(21) Application number: **21893935.3**

(22) Date of filing: **17.11.2021**

(51) International Patent Classification (IPC):
**A61K 31/00** (2006.01)   **A61P 25/00** (2006.01)
**C12N 15/58** (2006.01)

(52) Cooperative Patent Classification (CPC):
**A61K 31/00; A61P 25/00; C12N 9/6456;**
Y02A 50/30

(86) International application number:
**PCT/CN2021/131184**

(87) International publication number:
**WO 2022/105788 (27.05.2022 Gazette 2022/21)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **17.11.2020 PCT/CN2020/129460**

(71) Applicant: **Talengen International Limited
Hong Kong 999077 (HK)**

(72) Inventor: **LI, Jinan
Beijing 100089 (CN)**

(74) Representative: **Abel & Imray LLP
Westpoint Building
James Street West
Bath BA1 2DA (GB)**

Remarks:
The complete document including Reference
Table(s) and the Sequence Listing(s) can be
downloaded from the EPO website

(54) **METHOD AND DRUG FOR INCREASING BDNF LEVEL**

(57) The present invention relates to a method for promoting the formation of mature BDNF and increasing the level of BDNF. The method comprises administering a therapeutically effective amount of a plasminogen pathway activator to a subject. The present invention also relates to a pharmaceutical composition, a product and a kit which comprises the plasminogen pathway activator and are used for promoting the formation of mature BDNF and increasing the BDNF level.

## Description

TECHNICAL FIELD

The present application relates to a method for promoting the formation of mature BDNF and increasing the level of BDNF, comprising: administrating to a subject in need an effective amount of a component of plasminogen activation pathway or a compound related thereto, such as plasminogen, to repair injured nerves.

BACKGROUND ART

Brain-derived neurotrophic factor (BDNF) is a member of the neurotrophic factor (NTF) family. BDNF is mainly expressed in the central nervous system, mainly distributed in the hippocampus, amygdala and cortex, and also expressed in the heart, fat and skeletal muscle of the peripheral system. Tyrosine kinase receptor B (Trk B) is a specific high-affinity receptor for BDNF. BDNF can stimulate various signaling pathways by binding to Trk B, thereby exerting its special biological functions.

Brain-derived neurotrophic factor (BDNF) and its receptor tyrosine kinase receptor B (Trk B) gene mutation or functional loss can lead to energy metabolism imbalance in the body. BDNF plays an important role in learning and memory by regulating the survival, growth of neurons and maintaining their functions.

SUMMARY OF THE APPLICATION

The research of the present application finds that plasminogen pathway activators such as plasminogen can significantly promote the formation of mature BDNF and increase the level of BDNF, thereby playing a role in the survival, differentiation, growth and development of neurons.

In one aspect, the application relates to the following items:

1. A method for promoting the formation of mature BDNF and increasing BDNF level, comprising: administering to a subject a therapeutically effective amount of a plasminogen pathway activator.

2. The method according to item 1, wherein the plasminogen pathway activator promotes cleavage of Pro-BDNF to form mature BDNF and BDNF gene transcription or expression in the nerve tissue of the subject.

3. The method according to item 1, wherein the plasminogen pathway activator promotes the cleavage of Pro-BDNF to form mature BDNF and BDNF gene transcription and expression in the nerve tissue of a subject suffering from nerve tissue injury, wherein the nerve tissue injury is caused by one or more diseases or conditions selected from the group consisting of:

1) infection, selected from one or more of the following: meningitis, encephalitis, poliomyelitis and epidural abscess;
2) vascular diseases, selected from one or more of the following: stroke, transient ischemic attack (TIA), subarachnoid hemorrhage, subdural hemorrhage and hematoma, and epidural hemorrhage;
3) nerve structural injury diseases, selected from one or more of the following: brain or spinal cord injury, Bell palsy, cervical spondylosis, carpal tunnel syndrome, brain or spinal cord tumor, peripheral neuropathy and Guillain-Barré syndrome;
4) dysfunction, selected from one or more of the following: headache, epilepsy, insomnia, neuralgia, anxiety and depression;
5) neurodegenerative diseases, selected from one or more of the following: Alzheimer's disease, Parkinson's disease, Huntington's disease (HD), amyotrophic lateral sclerosis (ALS), multiple sclerosis (MS), spinocerebellar ataxia, and Pick disease;
6) motor neuron diseases, selected from one or more of the following: spinal muscular atrophy (SMA), progressive bulbar palsy, progressive muscle atrophy, and primary lateral sclerosis;
7) tumors, selected from one or more of the following: brain tumor and brain cancer.

4. The method according to item 1, wherein the plasminogen pathway activator promotes the cleavage of Pro-BDNF to form mature BDNF and BDNF gene transcription and expression in nerve tissue of a subject suffering from spinal muscular atrophy (SMA).
In some embodiments, the plasminogen pathway activator increases gene transcription, protein expression and level of an additional neurotrophic factor in the injured nerve tissue of a subject. In some embodiments, the neurotrophic factors comprise nerve growth factor (NGF), neurotrophic factor 3 (NT-3), neurotrophic factor 4/5 (NT-4/5), ciliary neurotrophic factor (CNTF), glial cell line-derived neurotrophic factor (GDNF), leukemia inhibitory factor (LIF), insulin-like growth factor-1 (IGF-1), transforming growth factor (TGF), epidermal growth factor (EGF), fibroblast growth factor (FGF) or platelet-derived growth factor (PDGF).
In some embodiments, the present application relates to a method for preventing or treating a BDNF-related disease or condition, comprising administering to a subject a therapeutically effective amount of a plasminogen pathway activator.
In some embodiments, the BDNF-related disease or condition comprises any one selected from the following:

1) infection, selected from any one of the following: meningitis, encephalitis, poliomyelitis and epidural abscess;

2) vascular diseases, selected from any one of the following: stroke, transient ischemic attack (TIA), subarachnoid hemorrhage, subdural hemorrhage and hematoma, and epidural hemorrhage;

3) nerve structural injury diseases, selected from any one of the following: brain or spinal cord injury, Bell palsy, cervical spondylosis, carpal tunnel syndrome, brain or spinal cord tumor, peripheral neuropathy and Guillain-Barré syndrome;

4) dysfunction, selected from any one of the following: headache, epilepsy, insomnia, neuralgia, anxiety and depression;

5) neurodegenerative diseases, selected from any of the following: Alzheimer's disease, Parkinson's disease, Huntington's disease (HD), amyotrophic lateral sclerosis (ALS), multiple sclerosis (MS), spinocerebellar ataxia and Pick disease;

6) motor neuron diseases, selected from any one of the following: spinal muscular atrophy (SMA), progressive bulbar palsy, progressive muscle atrophy, and primary lateral sclerosis;

7) tumors, selected from any one of the following: brain tumor and brain cancer;

8) peripheral neuropathy, nerve injury caused by trauma, optic neuropathy, polyneuritis, herpes zoster, facial paralysis, burn injury, bedsore, corneal ulcer, and side effects caused by radiotherapy or chemotherapy;

9) neuropsychiatric disorder, obsessive-compulsive disorder, post-traumatic stress disorder, anorexia nervosa, autoimmune diseases of the central nervous system, long-term or short-term memory disorder, children's learning disability, closed craniocerebral injury, attention deficit disorder, narcolepsy, sleep disorder, brain or nerve cell injury, and AIDS-related neurologic deficit, motor and convulsive disorder characterized by motor and/or vocal convulsion (for example, Tourette mental disorder, chronic motor or vocal convulsive disorder, short-term convulsive disorder and stereotypical movement disorder), substance abuse disorder (for example, substance dependence, substance abuse and sequelae of substance abuse/dependence, such as substance induced psychological disorder, substance withdrawal and substance induced dementia or amnesia), traumatic brain injury, tinnitus, ataxia, muscular rigidity (spasticity), neurotoxicity, mental retardation or cognitive impairment (e.g., non-syndromic X-linked mental retardation, fragile X syndrome, Down syndrome, autism) caused by alcohol or substance abuse (e.g., ecstasy, methamphetamine, etc.), aphasia, Bell palsy, Creutzfeldt-Jakob disease, encephalitis, age-related macular degeneration, ondine syndrome, WAGR syndrome, hearing loss, Reiter syndrome, optic nerve injury, diabetic neuropathy, complications of nerve transplantation, peripheral nerve injury, obesity, metabolic syndrome, asthma, atopic disease, allergic inflammation, eczema, neuroimmunologic disease or disorder, neuro otological disease or disorder, and aging and aging related disease or disorder;

10) neuralgia, any one selected from the group consisting of: trigeminal neuralgia, chronic pain, chronic inflammatory pain, pain related to arthritis, fibromyalgia, pain related to cancer, pain related to digestive diseases, pain related to Crohn's disease, pain related to autoimmune disease, pain related to endocrine disease, pain related to diabetes neuropathy, phantom limb pain, spontaneous pain, chronic postoperative pain, chronic temporomandibular pain, burning pain, post-herpetic neuralgia, AIDS-related pain, type I and II complex regional pain syndrome, chronic back pain, pain related to spinal cord injury, pain related to drug intake and recurrent acute pain, neuropathic pain

5. The method according to any one of items 1-4, wherein the plasminogen pathway activator increases the plasminogen level in the diseased nerve tissue of a subject.

6. The method according to any one of items 1-5, wherein the plasminogen pathway activator is administered in combination with one or more other drugs or therapies.

7. The method according to any one of items 1-6, wherein the plasminogen pathway activator is administered intravenously, intramuscularly, intrathecally, by nasal inhalation, aerosol inhalation, by nasal drop or eye drop.

8. The method according to any one of items 1-7, wherein the plasminogen pathway activator is a component of the plasminogen activation pathway, preferably plasminogen.

9. The method according to any one of items 1-8, wherein the plasminogen pathway activator has one or more uses or activities selected from the group consisting of: promoting the survival, differentiation, growth and development of neurons; preventing neurons from being injured and dying; promoting the regeneration and differentiation of injured neurons; and maintaining neuron survival and normal physiological function.

10. The method according to any one of items 1-9, wherein the plasminogen comprises an amino acid sequence having at least 80%, 85%, 90%, 95%, 96%, 97%, 98% or 99% sequence identity with the amino acid sequence represented by SEQ ID NO:

2, 6, 8, 10 or 12, and has plasminogen activity.

11. The method according to any one of items 1-9, wherein the plasminogen is an active fragment of plasminogen, and is a protein having proteolytic activity or lysine binding activity of plasminogen.

12. The method according to any one of items 1-9, wherein the plasminogen is selected from the group consisting of: Glu-plasminogen, Lys-plasminogen, mini-plasminogen, micro-plasminogen, delta-plasminogen, or a variant thereof retaining plasminogen activity.

13. The method according to any one of items 1-9, wherein the plasminogen comprises the amino acid sequence represented by SEQ ID NO: 2, 6, 8, 10 or 12.

In the above technical solutions, the plasminogen pathway activator is one or more selected from the group consisting of: a component of plasminogen activation pathway, a compound directly activating plasminogen or indirectly activating plasminogen by activating an upstream component of plasminogen activation pathway, a compound mimicking the activity of plasminogen or plasmin, a compound upregulating the expression of plasminogen or an activator of plasminogen, an analog of plasminogen, an analog of plasmin, an analog of tPA or uPA, and an antagonist of fibrinolysis inhibitor.

In some particular embodiments, the component of plasminogen activation pathway is selected from the group consisting of: natural or recombinant plasminogen, human plasmin, Lys-plasminogen, Glu-plasminogen, plasmin, a variant of plasminogen and plasmin and the analog thereof comprising one or more kringle domains and protease domains of plasminogen and plasmin, mini-plasminogen, mini-plasmin, micro-plasminogen, micro-plasmin, delta-plasminogen, delta-plasmin, an activator of plasminogen, tPA and uPA. In some particular embodiments, the antagonist of the fibrinolysis inhibitor is an antagonist of natural or recombinant PAI-1, complement C1 inhibitor, $\alpha$2 antiplasmin, or $\alpha$2 macroglobulin, such as an antibody of PAI-1, complement C1 inhibitor, $\alpha$2 antiplasmin, or $\alpha$2 macroglobulin.

In some particular embodiments, the plasminogen pathway activator is administered in combination with one or more other drugs or therapies, preferably, the therapies comprise cell therapy (e.g., stem cell therapy) and gene therapy, e.g., antisense RNA, small molecule splicing modifiers.

In some particular embodiments, the plasminogen pathway activator is a component of the plasminogen activation pathway, e.g., plasminogen. In some particular embodiments, the plasminogen comprises or has an amino acid sequence having at least 75%, 80%, 85%, 90%, 95%, 96%, 97%, 98% or 99% sequence identity with the amino acid sequence represented by SEQ ID NO: 2, 6, 8, 10 or 12, and has plasminogen activity and/or lysine binding activity. In some embodiments, the plasminogen is a protein with an addition, deletion, and/or substitution of 1-100, 1-90, 1-80, 1-70, 1-60, 1-50, 1-45, 1-40, 1-35, 1-30, 1-25, 1-20, 1-15, 1-10, 1-5, 1-4, 1-3, 1-2, or 1 amino acid(s) based on SEQ ID NO: 2, 6, 8, 10 or 12, and having plasminogen activity and/or lysine binding activity. In some particular embodiments, the plasminogen activity is the proteolytic activity of plasminogen. In some particular embodiments, the plasminogen is a protein comprising an active fragment of plasminogen and having plasminogen activity and/or lysine binding activity. In some particular embodiments, the plasminogen activity is the proteolytic activity of plasminogen. In some particular embodiments, the active fragment of plasminogen comprises or has a serine protease domain of plasminogen or a plasminogen protease domain. In some particular embodiments, the amino acid sequence of the active fragment of plasminogen is represented by SEQ ID NO: 14. In some particular embodiments, the plasminogen is selected from the group consisting of: Glu-plasminogen (human full-length plasminogen), Lys-plasminogen (a human full-length plasminogen after cleavage between amino acid positions 76-77), mini-plasminogen (comprising Kringle 5 (K5) and serine protease domain), micro-plasminogen (comprising serine protease domain), delta-plasminogen (comprising Kringle 1 and serine protease domain), or a variant thereof retaining plasminogen activity. In some particular embodiments, the plasminogen is a human full-length plasminogen, or a variant or fragment thereof that still retains plasminogen activity and/or lysine binding activity. In some embodiments, the plasminogen is a human plasminogen orthologue from a primate or a rodent, or a variant or fragment thereof still retaining plasminogen activity and/or lysine binding activity. In some embodiments, the plasminogen comprises the amino acid sequence represented by SEQ ID NO: 2, 6, 8, 10 or 12. In some embodiments, the plasminogen is human natural plasminogen.

In some embodiments, the present application also relates to the technical solutions of the following items:

1. In one aspect, the present application relates to a plasminogen pathway activator or a pharmaceutical composition comprising the plasminogen pathway activator, for promoting the formation of mature BDNF and/or increasing the level of BDNF in nerve tissue of a subject. In one aspect, the present application also relates to use of a plasminogen pathway activator in the preparation of a medicament for promoting the formation of mature BDNF and/or increasing the level of BDNF in the nerve tissue of a subject.

2. The plasminogen pathway activator, or the pharmaceutical composition comprising the plasminogen pathway activator, or use thereof according to item 1, wherein the plasminogen pathway activator promotes the cleavage of Pro-BDNF to form mature BDNF and BDNF gene transcription or expression in the nerve tissue of a subject.

3. The plasminogen pathway activator, or the pharmaceutical composition comprising the plasminogen pathway activator, or use thereof according to item 1 or 2, wherein the plasminogen pathway activator increases gene transcription, protein expression and level of an additional neurotrophic factor in the nerve tissue of the subject.

4. The plasminogen pathway activator, or the pharmaceutical composition comprising the plasminogen pathway activator, or use thereof according to item 3, wherein the additional neurotrophic factor comprises nerve growth factor (NGF), neurotrophic factor 3 (NT-3), neurotrophic factor 4/5 (NT-4/5), ciliary neurotrophic factor (CNTF), glial cell line-derived neurotrophic factor (GDNF), leukemia inhibitory factor (LIF), insulin-like growth factor-1 (IGF-1), transforming growth factor (TGF), epidermal growth factor (EGF), fibroblast growth factor (FGF) or platelet-derived growth factor (PDGF).

5. The plasminogen pathway activator, or the pharmaceutical composition comprising the plasminogen pathway activator, or use thereof according to any one of items 1-4, wherein the plasminogen pathway activator has one or more uses or activities selected from the group consisting of: promoting the survival, differentiation, growth and development of neurons; preventing neurons from being injured and dying; promoting the regeneration and differentiation of injured neurons; and maintaining neuron survival and normal physiological function.

6. The plasminogen pathway activator, or the pharmaceutical composition comprising the plasminogen pathway activator, or use thereof according to any one of items 1-5, wherein the subject is a subject suffering from nerve or brain tissue injury, and wherein the nerve or brain tissue injury is caused by one or more diseases or conditions selected from the group consisting of:

1) infection, selected from one or more of the following: meningitis, encephalitis, poliomyelitis and epidural abscess;
2) vascular diseases, selected from one or more of the following: stroke, transient ischemic attack (TIA), subarachnoid hemorrhage, subdural hemorrhage and hematoma, and epidural hemorrhage;
3) nerve structural injury diseases, selected from one or more of the following: brain or spinal cord injury, Bell palsy, cervical spondylosis, carpal tunnel syndrome, brain or spinal cord tumor, peripheral neuropathy and Guillain-Barré syndrome;
4) dysfunction, selected from one or more of the following: headache, epilepsy, insomnia, neuralgia, anxiety and depression;
5) neurodegenerative diseases, selected from one or more of the following: Alzheimer's disease, Parkinson's disease, Huntington's disease (HD), amyotrophic lateral sclerosis (ALS), multiple sclerosis (MS), spinocerebellar ataxia, and Pick disease;
6) motor neuron diseases, selected from one or more of the following: spinal muscular atrophy (SMA), progressive bulbar palsy, progressive muscle atrophy, and primary lateral sclerosis;
7) tumors, selected from one or more of the following: brain tumor and brain cancer.

7. A plasminogen pathway activator, or a pharmaceutical composition comprising the plasminogen pathway activator for preventing or treating a BDNF-related disease or condition, or use of the plasminogen pathway activator in the preparation of a medicament for preventing or treating a BDNF-related disease or condition.

8. The plasminogen pathway activator or pharmaceutical composition or use thereof according to item 7, wherein the BDNF-related disease or condition comprises any one selected from the group consisting of:

1) infection, selected from any one of the following: meningitis, encephalitis, poliomyelitis and epidural abscess;
2) vascular diseases, selected from any one of the following: stroke, transient ischemic attack (TIA), subarachnoid hemorrhage, subdural hemorrhage and hematoma, and epidural hemorrhage;
3) nerve structural injury diseases, selected from any one of the following: brain or spinal cord injury, Bell palsy, cervical spondylosis, carpal tunnel syndrome, brain or spinal cord tumor, peripheral neuropathy and Guillain-Barré syndrome;
4) dysfunction, selected from any one of the following: headache, epilepsy, insomnia, neuralgia, anxiety and depression;
5) neurodegenerative diseases, selected from any of the following: Alzheimer's disease, Parkinson's disease, Huntington's disease (HD), amyotrophic lateral sclerosis (ALS), multiple sclerosis (MS), spinocerebellar ataxia and Pick disease;
6) motor neuron diseases, selected from any one of the following: spinal muscular atrophy (SMA), progressive bulbar palsy, progressive muscle atrophy, and primary lateral sclerosis;
7) tumors, selected from any one of the following: brain tumor and brain cancer;
8) peripheral neuropathy, nerve injury caused by trauma, optic neuropathy, polyneuritis, herpes zoster, facial paralysis, burn injury, bedsore, corneal ulcer, and side effects caused by radiotherapy or chemotherapy;

9) neuropsychiatric disorder, obsessive-compulsive disorder, post-traumatic stress disorder, anorexia nervosa, autoimmune diseases of the central nervous system, long-term or short-term memory disorder, children's learning disability, closed craniocerebral injury, attention deficit disorder, narcolepsy, sleep disorder, brain or nerve cell injury, and AIDS-related neurologic deficit, motor and convulsive disorder characterized by motor and/or vocal convulsion (for example, Tourette mental disorder, chronic motor or vocal convulsive disorder, short-term convulsive disorder and stereotypical movement disorder), substance abuse disorder (for example, substance dependence, substance abuse and sequelae of substance abuse/dependence, such as substance induced psychological disorder, substance withdrawal and substance induced dementia or amnesia), traumatic brain injury, tinnitus, ataxia, muscular rigidity (spasticity), neurotoxicity, mental retardation or cognitive impairment(e.g., non-syndromic X-linked mental retardation, fragile X syndrome, Down syndrome, autism) caused by alcohol or substance abuse (e.g., ecstasy, methamphetamine, etc.), aphasia, Bell palsy, Creutzfeldt-Jakob disease, encephalitis, age-related macular degeneration, ondine syndrome, WAGR syndrome, hearing loss, Reiter syndrome, optic nerve injury, diabetic neuropathy, complications of nerve transplantation, peripheral nerve injury, obesity, metabolic syndrome, asthma, atopic disease, allergic inflammation, eczema, neuroimmunologic disease or disorder, neuro otological disease or disorder, and aging and aging related disease or disorder;

10) neuralgia, any one selected from the group consisting of: trigeminal neuralgia, chronic pain, chronic inflammatory pain, pain related to arthritis, fibromyalgia, pain related to cancer, pain related to digestive diseases, pain related to Crohn's disease, pain related to autoimmune disease, pain related to endocrine disease, pain related to diabetes neuropathy, phantom limb pain, spontaneous pain, chronic postoperative pain, chronic temporomandibular pain, burning pain, post-herpetic neuralgia, AIDS-related pain, type I and II complex regional pain syndrome, chronic back pain, pain related to spinal cord injury, pain related to drug intake and recurrent acute pain, neuropathic pain.

9. The plasminogen pathway activator, or the pharmaceutical composition comprising the plasminogen pathway activator, or use thereof according to any one of items 1-8, wherein the plasminogen pathway activator increases plasminogen level in nerve tissue of the subject.

10. The plasminogen pathway activator, or the pharmaceutical composition comprising the plasminogen pathway activator, or use thereof according to any one of items 1-9, wherein the plasminogen pathway activator is administered in combination with one or more other drugs or therapies.

11. The plasminogen pathway activator, or the pharmaceutical composition comprising the plasminogen pathway activator, or use thereof according to any one of items 1-10, wherein the plasminogen pathway activator is administered intravenously, intramuscularly, intrathecally, by nasal inhalation, aerosol inhalation, by nasal drop or eye drop.

12. The plasminogen pathway activator, or the pharmaceutical composition comprising the plasminogen pathway activator, or use thereof according to any one of items 1-11, wherein the plasminogen pathway activator is one or more selected from the group consisting of: a component of plasminogen activation pathway, a compound directly activating plasminogen or indirectly activating plasminogen by activating an upstream component of plasminogen activation pathway, a compound mimicking the activity of plasminogen or plasmin, a compound upregulating the expression of plasminogen or an activator of plasminogen, an analog of plasminogen, an analog of plasmin, an analog of tPA or uPA, and an antagonist of fibrinolysis inhibitor.

13. The plasminogen pathway activator, or the pharmaceutical composition comprising the plasminogen pathway activator, or use thereof according to any one of items 1-12, wherein the component of plasminogen activation pathway is selected from the group consisting of: natural or recombinant plasminogen, human plasmin, Lys-plasminogen, Glu-plasminogen, plasmin, a variant of plasminogen and plasmin and the analog thereof comprising one or more kringle domains and/or protease domains of plasminogen and plasmin, mini-plasminogen, mini-plasmin, micro-plasminogen, micro-plasmin, delta-plasminogen, delta-plasmin, an activator of plasminogen, tPA and uPA.

14. The plasminogen pathway activator, or the pharmaceutical composition comprising the plasminogen pathway activator, or use thereof according to any one of items 1-12, wherein the antagonist of fibrinolysis inhibitor is an antagonist of natural or recombinant PAI-1, complement C1 inhibitor, $\alpha$2 antiplasmin or $\alpha$2 macroglobulin, e.g., an antibody of PAI-1, complement C1 inhibitor, $\alpha$2 antiplasmin or $\alpha$2 macroglobulin.

15. The plasminogen pathway activator, or the pharmaceutical composition comprising the plasminogen pathway activator, or use thereof according to any one of items 1-13, wherein the plasminogen pathway activator is plasminogen.

16. The plasminogen pathway activator, or the pharmaceutical composition comprising the plasminogen pathway activator, or use thereof according to item 15, wherein the plasminogen comprises the amino acid sequence represented by SEQ ID NO: 2, 6, 8, 10 or 12; or comprises an amino acid sequence having at least 80%, 85%, 90%, 95%, 96%, 97%, 98% or 99% sequence identity with the amino acid sequence represented by SEQ ID NO: 2, 6, 8, 10 or 12, and has the proteolytic activity and/or lysine binding activity of plasminogen.

17. The plasminogen pathway activator, or the pharmaceutical composition comprising the plasminogen pathway activator, or use thereof according to item 15, wherein the plasminogen comprises one or more selected from the group consisting of:

1) a serine protease domain having the amino acid sequence represented by SEQ ID NO: 14; 2) a serine protease domain having at least 80%, 90%, 95%, 96%, 97%, 98%, 99% identity with SEQ ID NO: 14 and retaining proteolytic activity; 3) a Kringle domain selected from one or more of Kringle 1, Kringle 2, Kringle 3, Kringle 4 and Kringle 5; and 4) a Kringle domain having at least 80%, 90%, 95%, 96%, 97%, 98%, 99% identity with one or more selected from Kringle 1, Kringle 2, Kringle 3, Kringle 4 and Kringle 5, and retaining lysine-binding activity.

18. The plasminogen pathway activator, or the pharmaceutical composition comprising the plasminogen pathway activator, or use thereof according to item 15, wherein the plasminogen is selected from Glu-plasminogen, Lys-plasminogen, mini-plasminogen, micro-plasminogen, or delta-plasminogen, or a variant thereof retaining the proteolytic activity of plasminogen.

In some particular embodiments, the plasminogen pathway activator is administered systemically or locally, e.g., administered by intravenous, intramuscular, intrathecal, or by nasal inhalation, aerosol inhalation, by nasal drop or eye drop. In some embodiments, the subject is a human. In some embodiments, the subject is deficient or absent of plasminogen. In some embodiments, the deficiency or absence is congenital, secondary and/or local. In some embodiments, the plasminogen is administrated daily, or every second day or every third day consecutively at a dose of 0.0001-2000 mg/kg, 0.001-800 mg/kg, 0.01-600 mg/kg, 0.1-400 mg/kg, 1-200 mg/kg, 1-100 mg/kg, 10-100mg/kg (calculating by per kilogram of body weight); or 0.0001-2000 mg/cm$^2$, 0.001-800 mg/cm$^2$, 0.01-600 mg/cm$^2$, 0.1-400 mg/cm$^2$, 1-200 mg/cm$^2$, 1-100 mg/cm$^2$, 10-100 mg/cm$^2$ (calculating by per square centimeter of body surface area).

In one aspect, the present application also relates to a pharmaceutical composition, medicament, preparation, kit, and product used in the above method, which comprises the above plasminogen pathway activator, e.g., the above plasminogen.

In some embodiments, the pharmaceutical composition, medicament, preparation comprises a pharmaceutically acceptable carrier and a plasminogen pathway activator, for example, a component of the plasminogen activation pathway, e.g., plasminogen. In some embodiments, the kit and product comprises one or more containers containing the pharmaceutical composition, medicament or preparation therein. In some embodiments, the kit or product further comprises a label or instructions for use indicating that the plasminogen pathway activator, for example, a component of the plasminogen activation pathway, e.g., plasminogen is used in the above method. In some embodiments, the kit or product further comprises one or more additional containers containing one or more additional drugs.

In one aspect, the present application also relates to a plasminogen pathway activator, such as plasminogen as described above, for the use as described above.

In one aspect, the present application also relates to use of the therapeutically effective amount of the above plasminogen pathway activator in the preparation of a pharmaceutical composition, medicament, preparation, kit, and product for the above methods.

In some embodiments, the plasminogen pathway activator is one or more selected from the group consisting of: a component of plasminogen activation pathway, a compound directly activating plasminogen or indirectly activating plasminogen by activating an upstream component of plasminogen activation pathway, a compound mimicking the activity of plasminogen or plasmin, a compound upregulating the expression of plasminogen or an activator of plasminogen, an analog of plasminogen, an analog of plasmin, an analog of tPA or uPA, and an antagonist of fibrinolysis inhibitor.

In some particular embodiments, the component of plasminogen activation pathway is selected from the group consisting of: plasminogen, recombinant human plasmin, Lys-plasminogen, Glu-plasminogen, plasmin, a variant of plasminogen and plasmin and the analog thereof comprising one or more kringle domains and/or protease domains of plasminogen and plasmin, mini-plasminogen, mini-plasmin, micro-plasminogen, micro-plasmin, delta-plasminogen, delta-plasmin, an activator of plasminogen, tPA and uPA. In some particular embodiments, the antagonist of fibrinolysis inhibitor is an antagonist of PAI-1, complement C1 inhibitor, α2 antiplasmin or α2 macroglobulin, e.g., an antibody of PAI-1, complement C1 inhibitor, α2 antiplasmin or α2 macroglobulin.

In some particular embodiments, the plasminogen pathway activator is administered in combination with one or more other drugs and/or therapies, preferably, the therapies comprise cell therapy (e.g., stem cell therapy) and gene therapy, such as antisense RNA, small molecule splicing modifiers.

In some particular embodiments, the plasminogen pathway activator is a component of the plasminogen activation pathway, e.g., plasminogen. In some particular embodiments, the plasminogen comprises or has an amino acid sequence having at least 75%, 80%, 85%, 90%, 95%, 96%, 97%, 98% or 99% sequence identity with the amino acid sequence represented by SEQ ID NO: 2, 6, 8, 10 or 12, and has the plasminogen activity and/or lysine binding activity. In some embodiments, the plasminogen is a protein with an addition, deletion, and/or substitution of 1-100, 1-90, 1-80, 1-70, 1-60, 1-50, 1-45, 1-40, 1-35, 1-30, 1-25, 1-20, 1-15, 1-10, 1-5, 1-4, 1-3, 1-2, or 1 amino acid(s) based on SEQ ID NO: 2, 6, 8, 10 or 12, and having plasminogen activity and/or lysine binding activity. In some particular embodiments, the plasminogen activity is the proteolytic activity of plasminogen. In some particular embodiments, the plasminogen is a protein comprising an active fragment of plasminogen and having plasminogen activity and/or lysine binding activity. In some particular embodiments, the plasminogen activity is the proteolytic activity of plasminogen. In some particular embodiments, the active fragment of plasminogen comprises or has a serine protease domain of plasminogen or a plasminogen protease domain. In some particular embodiments, the amino acid sequence of the active fragment of plasminogen is represented by SEQ ID NO: 14. In some particular embodiments, the plasminogen is selected from the group consisting of: Glu-plasminogen (human full-length plasminogen), Lys-plasminogen (a human full-length plasminogen after cleavage between amino acid positions 76-77), mini-plasminogen (comprising Kringle 5 (K5) and serine protease domain), micro-plasminogen (comprising serine protease domain), delta-plasminogen (comprising Kringle 1 and serine protease domain), or a variant thereof retaining plasminogen activity. In some particular embodiments, the plasminogen is human full-length plasminogen, or a variant or fragment thereof that still retains plasminogen activity and/or lysine binding activity. In some embodiments, the plasminogen is a human plasminogen orthologue from a primate or a rodent, or a variant or fragment thereof still retaining plasminogen activity and/or lysine binding activity. In some embodiments, the plasminogen comprises the amino acid sequence represented by SEQ ID NO: 2, 6, 8, 10 or 12. In some embodiments, the plasminogen is human natural plasminogen.

In some embodiments, the plasminogen pathway activator, for example, a component of the plasminogen activation pathway, e.g., plasminogen, is administered in combination with one or more other drugs and/or therapies. In some embodiments, the plasminogen pathway activator, for example, a component of the plasminogen activation pathway, e.g. plasminogen is administered intravenously, intramuscularly, intrathecally, by nasal inhalation, aerosol inhalation, by nasal drop or eye drop.

In some embodiments, the pharmaceutical composition, medicament, preparation comprises a pharmaceutically acceptable carrier and a plasminogen pathway activator, for example, a component of the plasminogen activation pathway, e.g., plasminogen. In some embodiments, the kit and product comprises one or more containers containing the pharmaceutical composition, medicament or preparation therein. In some embodiments, the kit or product further comprises a label or instructions for use indicating that the plasminogen pathway activator, for example, a component of the plasminogen activation pathway, e.g., plasminogen is used in the above method.

In some embodiments, the kit or product further comprises one or more additional containers containing one or more additional drugs.

The present application explicitly encompasses all the combinations of the technical features belonging to the embodiments of the present application, and these combined technical solutions have been explicitly disclosed in this application, just as the separately and explicitly disclosed above technical solutions. In addition, the present application also explicitly encompasses the combinations of each embodiment and its elements, and the combined technical solutions are explicitly disclosed herein.

BRIEF DESCRIPTION OF THE DRAWINGS

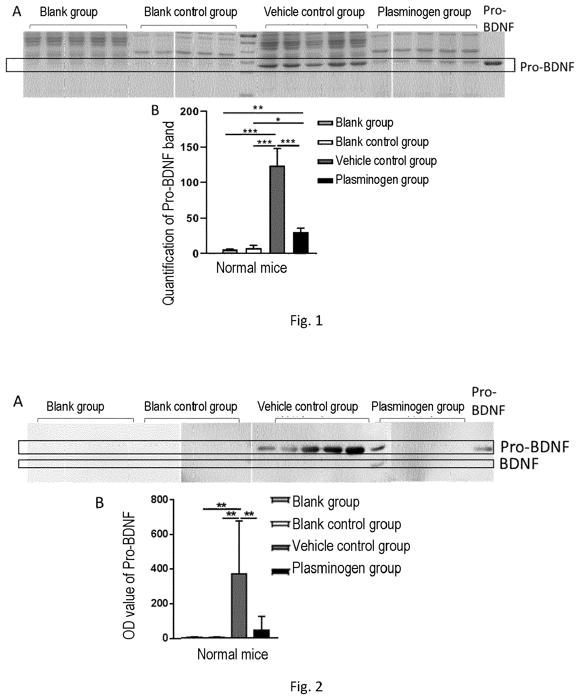

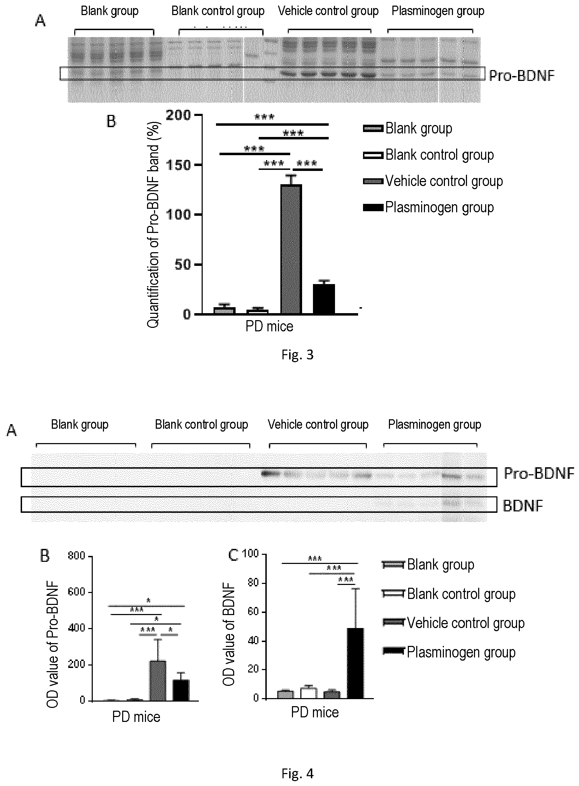

Figs. 1A-1B show the effect of plasminogen on recombinant human Pro-BDNF in brain homogenate of normal mice. Fig. 1A is an image of SDS-PAGE imaging, Fig. 1B is the result of quantitative analysis of SDS-PAGE bands. The results show that in brain homogenate of normal mice, the amount of Pro-BDNF in the plasminogen administration group is significantly lower than that in the vehicle control group, and the difference is extremely significant (*** represents P<0.001). It is suggested that plasminogen can promote the cleavage of recombinant human Pro-BDNF in brain homogenate of normal mice. Figs.2A-2B show the effect of plasminogen on recombinant human Pro-BDNF in brain homogenate of normal mice. Fig. 2A is an image of Western blot imaging, and Fig. 2B is the analysis result of optical density (OD) value of Pro-BDNF band in Western blot. The results show that in the brain homogenate of normal mice, the amount of Pro-BDNF in the plasminogen administration group is significantly lower than that in the vehicle control group, and the difference is extremely significant (** represents P<0.01). It is suggested that plasminogen can promote the cleavage of recombinant human Pro-BDNF in brain homogenate of normal mice. Figs. 3A-3B show the effect of plasminogen on recombinant human Pro-BDNF in the brain homogenate of Parkinson's disease model mice. Fig.3A is an image of SDS-PAGE imaging, and Fig.3B is the result of quantitative analysis of SDS-PAGE bands.

The results show that in the brain homogenate of Parkinson's disease model mice, the amount of Pro-BDNF in the plasminogen administration group is significantly lower than that in the vehicle control group, and the difference is extremely significant (*** represents P<0.001). It is suggested that plasminogen can promote the cleavage of recombinant human Pro-BDNF in brain homogenate of Parkinson's disease model mice.

Figs. 4A-4C show the effect of plasminogen on recombinant human Pro-BDNF in brain homogenate of Parkinson's disease model mice. Fig. 4A is an image of Western blot imaging, Fig. 4B is the analysis result of optical density (OD) value of Pro-BDNF band in Western blot, Fig. 4C is the analysis result of optical density (OD) value of BDNF band in Western blot. The results show that in the brain homogenate of Parkinson's disease model mice, the amount of Pro-BDNF in the plasminogen administration group is significantly lower than that in the vehicle control group, and the difference is significant (* represents P<0.05, *** represents P<0.001 ); the amount of BDNF in the plasminogen administration group is significantly higher than that in the vehicle control group, and the difference is extremely significant. It is suggested that plasminogen can promote the cleavage of recombinant human Pro-BDNF and the formation of mature BDNF in brain homogenate of Parkinson's disease model mice.

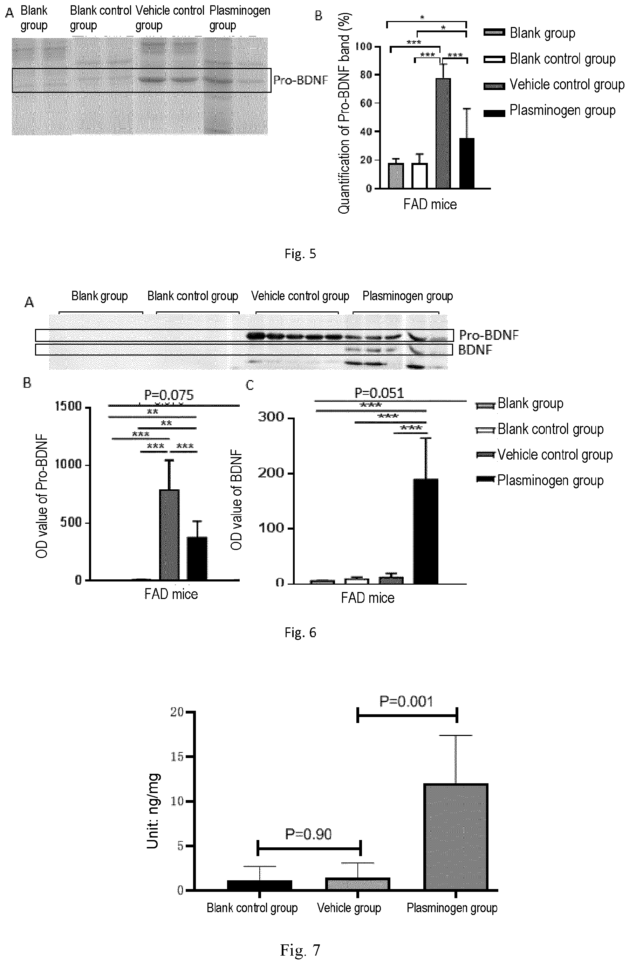

Figs. 5A-5B show the effect of plasminogen on recombinant human Pro-BDNF in the brain homogenate of Alzheimer's model mice. Fig. 5A is an image of SDS-PAGE imaging, and Fig. 5B is the result of quantitative analysis of Pro-BDNF bands in SDS-PAGE. The results show that in the brain homogenate of Alzheimer's disease model mice, the amount of Pro-BDNF in the plasminogen administration group is significantly lower than that in the vehicle control group, and the difference is extremely significant (*** represents P<0.001). It is suggested that plasminogen can promote the cleavage of Pro-BDNF in brain homogenate of Alzheimer's disease model mice.

Figs. 6A-6C show the effect of plasminogen on recombinant human Pro-BDNF in the brain homogenate of Alzheimer's model mice. Fig. 6A is an image of Western blot imaging, Fig. 6B is the analysis result of optical density (OD) value of Pro-BDNF band in Western blot, and Fig. 6C is the analysis result of optical density (OD) value of BDNF band in Western blot. The results show that in the brain homogenate of Alzheimer's disease model mice, the amount of Pro-BDNF in the plasminogen group is significantly lower than that in the vehicle control group, and the difference is extremely significant (** indicates P<0.01, *** indicates P<0.001); the amount of BDNF in the plasminogen group is significantly higher than that in the vehicle control group, and the difference is significant. It is suggested that plasminogen can promote the cleavage of Pro-BDNF and the formation of mature BDNF in the brain homogenate of Alzheimer's disease model mice.

Fig. 7 shows the detection results of the content of plasminogen in the brain tissue of SMNΔ7 SMA mice by ELISA, after continuous administration of plasminogen. The results show that the plasminogen level in the brain tissue of wild-type mice in the blank control group is 1. 18±1.54ng/mg; the plasminogen level in the brain tissue of mice in the vehicle group is 1.49±1.59ng/mg, the plasminogen level in the brain tissue of mice in the vehicle group did not change significantly as compared with the blank control group; the plasminogen level in SMA transgenic mice in the plasminogen group is 12.09±5.32ng/mg, about 8 times that of the vehicle group. It is suggested that supplementing plasminogen can promote the permeability of blood-brain barrier to plasminogen under SMA disease conditions, and plasminogen can pass through the blood-brain barrier and reach the brain.

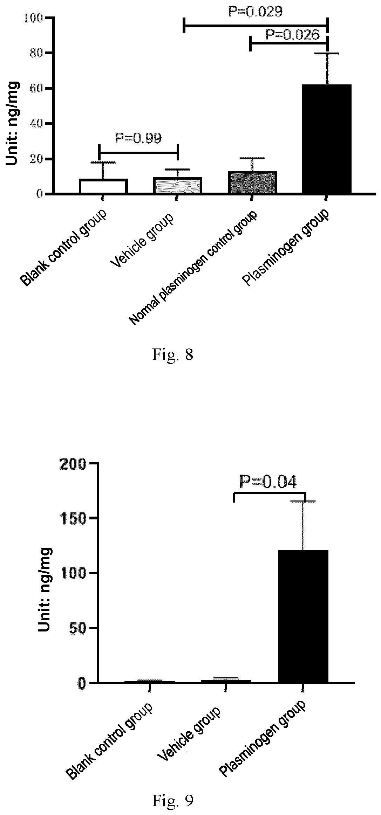

Fig. 8 shows the detection results of the content of plasminogen in SMNΔ7 SMA spinal cord tissue by ELISA, after continuous administration of plasminogen. The results show that the plasminogen level in the spinal cord of wild-type mice in the blank control group is 8.51±9.51ng/mg. The plasminogen level in the spinal cord of SMA transgenic mice in the vehicle group is 19.95±4.06ng/mg, which is slightly higher than that in the blank control group. The plasminogen level in the normal plasminogen administration control group is 13.03±7.51ng/mg. The plasminogen level in the spinal cord of the SMA transgenic mice in the plasminogen group is 62.33±17.37ng/mg, which is about three times that of the mice in the vehicle group, and the statistical analysis P value as compared between the plasminogen group and the vehicle group is 0.029, which is 4.8 times that of the normal plasminogen administration control group, and the statistical analysis P value as compared between the plasminogen group and the normal plasminogen administration control group is 0.026. It is suggested that the administration of plasminogen can promote the increase of the permeability of the blood-spinal cord barrier to plasminogen under the condition of SMA, and the plasminogen can pass through the blood-spinal cord barrier and reach the spinal cord.

Fig. 9 shows the detection results of plasminogen level in spinal cord homogenate by ELISA, 2 hours after a single tail vein injection of plasminogen in LPS-induced pneumonia SMA heterozygous mice. The results show that the plasminogen level in spinal cord tissue homogenate of mice in the blank control group is 2.70±0.74ng/mg; after tracheal instillation of LPS, the plasminogen level in spinal cord tissue homogenate of mice in vehicle group is 3.17±1.51ng/mg, and there is no significant change as compared with the blank control group; after the mice in the plasminogen group are supplemented with 2.5 times the physiological dose of plasminogen, the plasminogen level is 121.16±44.68ng/mg, which is about 38.2 times that of the mice in the vehicle group. It is suggested that supplementing plasminogen can promote the permeability of blood-spinal cord barrier to plasminogen in LPS-induced pneumonia SMA heterozygous mice. Under this condition, plasminogen can pass through the blood-spinal cord barrier to enter the central nervous system.

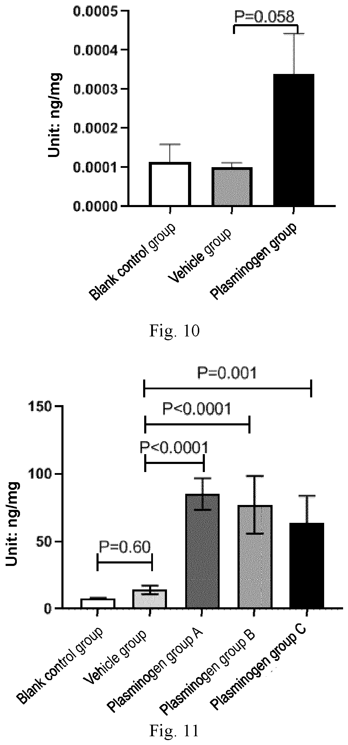

Fig. 10 shows the detection results of plasminogen level in spinal cord homogenate by enzyme-substrate kinetics method, 2 hours after a single tail vein injection of plasminogen in LPS-induced pneumonia SMA heterozygous mice. The results show that the plasminogen level in spinal cord tissue of mice in the blank control group is $0.00011±4.51×10^{-5}$ U/mg; after tracheal instillation of LPS, the plasminogen level in the spinal cord of mice in the vehicle group is $0.00010±9.72×10^{-6}$ U/mg, and there is no significant change as compared with the blank control group; after the mice in the plasminogen group are supplemented with 2.5 times the physiological dose of plasminogen, the plasminogen level is increased to $0.00034±1.04×10^{-4}$ U/mg, the statistical analysis P value is 0.058 as compared with that of the vehicle group. It is suggested that supplementing plasminogen can promote the permeability of blood-spinal cord barrier to plasminogen in LPS-induced pneumonia SMA heterozygous mice, and plasminogen can pass through the blood-spinal cord barrier to accumulate in the spinal cord.

Fig. 11 shows the detection results of plasminogen level in spinal cord homogenate by ELISA, 2 hours after a single tail vein injection of plasminogen to LPS-induced pneumonia mice. The results of ELISA detection of plasminogen level in the spinal cord tissue homogenate show that, the Plg content of the spinal cord tissue in the blank control group is 7.71±0.51ng/mg, and the Plg content of the spinal cord tissue of the mice in the vehicle group is 14.04±3.25ng/mg. After tracheal instillation of LPS, the plasminogen level in the spinal cord of the mice is increased; after supplementing 50 mg/kg (about 1 mg per mouse) of plasminogen to the mice in plasminogen group A, the plasminogen level in the spinal cord tissue is 85.10 ± 11.59 ng/mg, which is about 6.1 times that of the mice in the vehicle group; after supplementing 17mg/kg (about 0.34mg per mouse) of plasminogen to the mice in plasminogen group B, the plasminogen level in spinal cord tissue is 77.90± 21.39ng/mg, which is about 5.5 times that of the mice in the vehicle group; after the mice in the plasminogen group C are supplemented with plasminogen at 6mg/kg (about 0.1mg per mouse), the plasminogen level in spinal cord tissue is 64.00 ±19.63ng/mg, which is about 4.6 times that of the mice in the vehicle group. It is suggested that: 1) supplementing plasminogen can promote the permeability of blood-brain barrier to plasminogen in LPS-induced pneumonia mice, and plasminogen can pass through the blood-brain barrier to enrich in spinal cord tissue; 2) the enrichment of plasminogen in the spinal cord has a dose-dependent effect, within the dose range of 6mg-50mg/kg, the enrichment plasminogen level in the spinal cord increases with the increase of the administration dose of plasminogen.

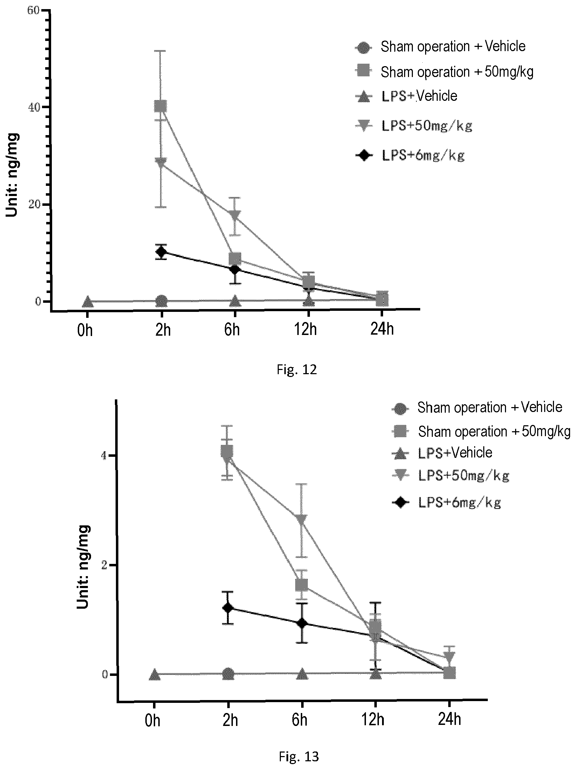

Fig. 12 shows detection results of human plasminogen level in spinal cord tissue homogenate by ELISA, at different time points after tail vein injection of plasminogen into C57 pneumonia model mice. The results show that mice in the sham operation+vehicle group and LPS+vehicle group do not receive plasminogen injection, and no human plasminogen is detected in the spinal cord tissue homogenate at 0, 2, 6, 12 and 24 hours. Mice in the sham operation+50mg/kg plasminogen group and LPS+50mg/kg plasminogen group are received 50mg/kg body weight plasminogen injection, and human plasminogen in all the spinal cord tissue homogenate is increased significantly 2 hours after injection, and the plasminogen level is decreased significantly 6-12 hours after injection, and no human plasminogen is basically detected at 24 hours. Mice in the LPS+6mg/kg plasminogen group are received 6mg/kg body weight plasminogen injection, and 2 hours after injection, the plasminogen level detected in spinal cord tissue homogenate is significantly higher than that of LPS+vehicle group mice, and significantly lower than that of LPS+50mg/kg plasminogen group mice. The results indicate that: 1) after administration of plasminogen under physiological and pathological conditions, plasminogen can be promoted to pass through the blood-brain barrier to enrich in spinal cord tissue; 2) intravenous injection of plasminogen in normal mice *in vivo,* the plasminogen level in spinal cord tissue is increased significantly; 3) the enrichment of plasminogen in the spinal cord has a time-dependent effect, which is increased firstly, then gradually decreased in 2-12 hours, and is almost completely metabolized in 12-24 hours; 4) the enrichment of plasminogen in the spinal cord has a dose-dependent effect, the higher the administration dose, the higher the plasminogen level in the spinal cord tissue.

Fig. 13 shows detection results of plasminogen level in brain tissue homogenate by ELISA, at different time points after tail vein injection of plasminogen into C57 pneumonia model mice. The results show that mice in the blank+vehicle group and LPS+vehicle group do not receive plasminogen injection, and no human plasminogen is detected in the brain tissue homogenate at 0, 2, 6, 12 and 24 hours. Mice in blank+50mg/kg plasminogen group and LPS+50mg/kg plasminogen group are received 50mg/kg body weight plasminogen injection, human plasminogen detected is increased significantly in the brain tissue homogenate 2 hours after injection, and the plasminogen level is decreased significantly 6-12 hours after injection, and no human plasminogen is basically detected at 24 hours. Mice in the LPS+6mg/kg plasminogen group are received 6mg/kg body weight plasminogen injection, and the plasminogen level detected in brain tissue homogenate 2 hours after injection is significantly higher than that of LPS+vehicle group mice, and significantly lower than that of LPS+50mg/kg plasminogen group mice. The results indicate that: 1) after administration of plasminogen under physiological and pathological conditions, it can promote plasminogen to pass through the blood-brain barrier to enrich in brain tissue; 2) intravenous injection of plasminogen in normal mice *in vivo,* the plasminogen level in brain tissue is increased significantly; 3) the enrichment of plasminogen in brain tissue has a time-dependent effect, which is increased firstly, then gradually decreased in 2-12 hours, and is almost completely metabolized in 12-24 hours; 4) the enrichment of plasminogen in brain tissue has a dose-dependent effect, the higher the administration dose, the higher the plasminogen level in brain tissue.

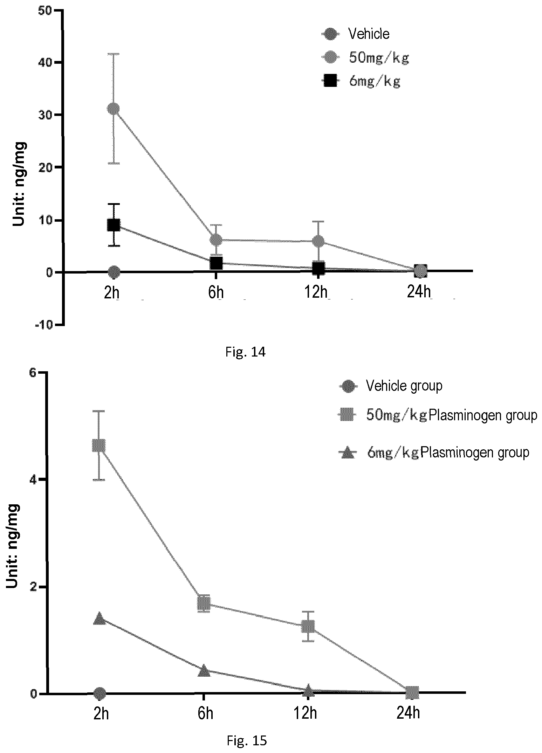

Fig. 14 shows detection results of human plasminogen level in spinal cord tissue homogenate by ELISA, at different time points after tail vein injection of plasminogen into SOD1-G93A mice. The results of ELISA level detection of the spinal cord of SOD1-G93A mice show that, the plasminogen level in the spinal cord of SOD1-G93A mice is significantly increased after tail vein injection of 50 mg/kg and 6 mg/kg plasminogen, and the plasminogen level in 50 mg/kg plasminogen group is significantly higher than that of mice in the 6mg/kg group. The plasminogen level is gradually decreased 2 hours after administration, and its metabolism is basically completely finished in 12-24 hours.

The results indicate that: 1) administration of plasminogen at a physiological dose level, plasminogen can pass through the blood-brain barrier of SOD1-G93A mice to enrich in the spinal cord tissue; 2) the enrichment of plasminogen in the spinal cord has a dose-dependent effect, and the higher the administration dose of plasminogen, the more enriched; 3) the enrichment of plasminogen in the spinal cord has a time-dependent effect, which is increased firstly, then gradually decreased in 2-12 hours, and its metabolism is basically completely finished in 12-24 hours.

Fig. 15 shows detection results of plasminogen level in brain tissue homogenate by ELISA, at different time points after tail vein injection of plasminogen into SOD1-G93A mice. The results of ELISA level detection of the brain of SOD1-G93A mice show that, the plasminogen level in the brain tissue of SOD1-G93A mice is significantly increased after tail vein injection of 50 mg/kg and 6 mg/kg plasminogen, and the plasminogen level in the 50 mg/kg plasminogen group is significantly higher than that of mice in the 6mg/kg plasminogen group. The plasminogen level is gradually decreased 2 hours after administration, and its metabolism is basically completely finished in 12-24 hours. The results indicate that: 1) after administration of plasminogen at a physiological dose level it can pass through the blood-brain barrier of SOD1-G93A mice to enrich in brain tissue; 2) the enrichment of plasminogen in brain tissue has a dose-dependent effect, the higher the administration dose of plasminogen, the more enriched; 3) the enrichment of plasminogen in the brain tissue has a time-dependent effect, which is increased firstly, then decreased gradually in 2-12 hours, and its metabolism is basically completely finished in 12-24 hours.

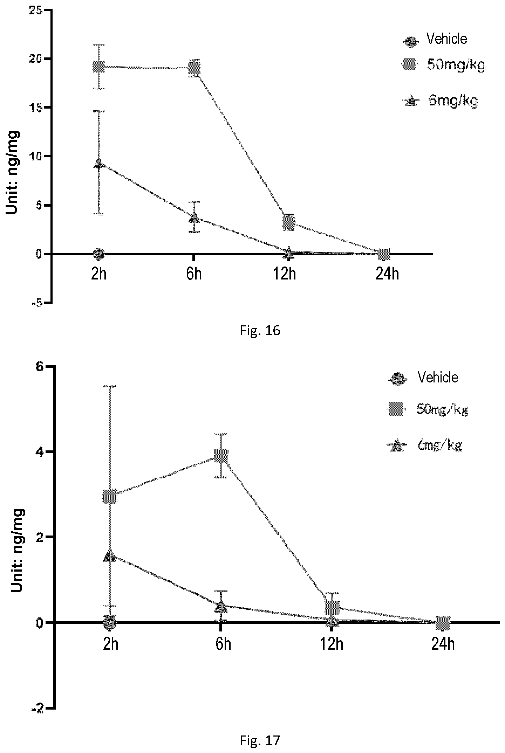

Fig. 16 shows detection results of plasminogen level in spinal cord tissue homogenate by ELISA, at different time points after tail vein injection of plasminogen into FAD mice. The results of ELISA level detection of the spinal cord tissue homogenate show that, the plasminogen level in the spinal cord tissue of FAD mice is significantly increased after tail vein injection of 50 mg/kg and 6 mg/kg plasminogen, and the plasminogen level in the 50 mg/kg plasminogen group is significantly higher than that of mice in the 6mg/kg plasminogen group. The plasminogen level is gradually decreased 2 hours after administration, and its metabolism is basically completely finished in 12-24 hours. The results indicate that: 1) after administration of plasminogen at a physiological dose level it can pass through the blood-brain barrier of FAD mice to enrich in spinal cord tissue; 2) the enrichment of plasminogen in spinal cord tissue has a dose-dependent effect, the higher the administration dose of plasminogen, the more enriched; 3) the enrichment of plasminogen in the spinal cord tissue has a time-dependent effect, which is increased firstly, then decreased gradually in 2-12 hours, and its metabolism is basically completely finished in 12-24 hours.

Fig. 17 shows detection results of plasminogen level in brain tissue homogenate by ELISA, at different time points after tail vein injection of plasminogen into FAD mice. The results of ELISA level detection of the brain tissue homogenate show that, the plasminogen level in the brain tissue of FAD mice is significantly increased after tail vein injection of 50 mg/kg and 6 mg/kg plasminogen, and the plasminogen level in the 50 mg/kg plasminogen group is significantly higher than that of mice in the 6mg/kg plasminogen group. The plasminogen level is gradually decreased 2 hours after administration, and its metabolism is basically completely finished in 12-24 hours. The results indicate that: 1) after administration of plasminogen at a physiological dose level it can pass through the blood-brain barrier of FAD mice to enrich in brain tissue; 2) the enrichment of plasminogen in brain tissue has a dose-dependent effect, the higher the administration dose of plasminogen, the more enriched; 3) the enrichment of plasminogen in the brain tissue has a time-dependent effect, which is increased firstly, then decreased gradually in 2-12 hours, and its metabolism is basically completely finished in 12-24 hours.

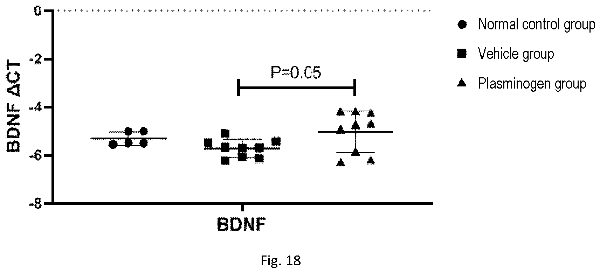

Fig. 18 shows PCR detection of BDNF gene mRNA in spinal cord tissue of SOD1-G93A mice after tail vein injection of plasminogen. The results show that the transcription level of BDNF gene in the spinal cord tissue of mice in the plasminogen group is significantly higher than that in the vehicle group, and the statistical difference P value is 0.05. It is suggested that plasminogen can promote the transcription of BDNF gene in the spinal cord of SOD1-G93A mice.

## DETAILED DESCRIPTION OF THE APPLICATION

Fibrinolytic system is a system consisting of a series of chemical substances involved in the process of fibrinolysis, mainly including plasminogen, plasmin, plasminogen activator, and fibrinolysis inhibitor. Plasminogen activators include tissue-type plasminogen activator (t-PA) and urokinase-type plasminogen activator (u-PA). t-PA is a serine protease that is synthesized by vascular endothelial cells. t-PA activates plasminogen, which is mainly carried out on fibrin; urokinase-type plasminogen activator (u-PA) is produced by renal tubular epithelial cells and vascular endothelial cells, and may directly activate plasminogen without the need for fibrin as a cofactor. Plasminogen (PLG) is synthesized by the liver. When blood coagulates, a large amount of PLG is adsorbed on the fibrin network, and under the action of t-PA or u-PA it is activated into plasmin to promote fibrinolysis. Plasmin (PL) is a serine protease whose functions are as follows: degrading fibrin and fibrinogen; hydrolyzing various coagulation factors V, VIII, X, VII, XI, and II, etc.; converting plasminogen into plasmin; hydrolyzing complement, etc. Fibrinolysis inhibitors: including plasminogen activator inhibitor (PAI) and α2 antiplasmin (α2-AP). PAI mainly has two forms, PAI-1 and PAI-2, which may specifically bind to t-PA in a ratio of 1:1, thereby inactivating it and activating PLG at the same time. α2-AP is synthesized by the liver, and binds to PL in a ratio of 1:1 to form a complex to inhibit the activity of PL; FXIII makes α2-AP covalently bound to fibrin, reducing the sensitivity of fibrin to PL. Substances that inhibit the activity of the fibrinolytic system *in vivo*: PAI-1, complement C1 inhibitor; α2 antiplasmin; α2 macroglobulin.

The term "plasminogen pathway activator" herein encompasses a component of plasminogen activation pathway, a compound capable of directly activating plasminogen or indirectly activating plasminogen by activating a upstream component of plasminogen activation pathway, a compound mimicking the activity of plasminogen or plasmin, a compound upregulating the expression of plasminogen or an activator of plasminogen, an analog of plasminogen, an analog of plasmin, an analog of tPA or uPA, and an antagonist of fibrinolysis inhibitor.

The term "component of plasminogen activation pathway" according to the present application encompasses:

    1. plasminogen, Lys-plasminogen, Glu-plasminogen, micro-plasminogen, delta-plasminogen; variants or analogs thereof;
    2. plasmin and a variant or analog thereof; and
    3. plasminogen activators, such as tPA and uPA, and tPA or uPA variants and analogs comprising one or more domains of tPA or uPA, such as one or more kringle domains and proteolytic domains.

The term "an antagonist of a fibrinolytic inhibitor" encompasses an antagonist of PAI-1, complement C1 inhibitor, α2 antiplasmin or α2 macroglobulin, such as an antibody of PAI-1, complement C1 inhibitor, α2 antiplasmin or α2 macroglobulin.

"Variants" of the above plasminogen, plasmin, tPA and uPA include all naturally occurring human genetic variants as well as other mammalian forms of these proteins, as well as a protein obtained by addition, deletion and/or substitution of such as 1-100, 1-90, 1-80, 1-70, 1-60, 1-50, 1-45, 1-40, 1-35, 1-30, 1-25, 1-20, 1-15, 1-10, 1-5, 1-4, 1-3, 1-2, or 1 amino acid, and still retaining the activity of plasminogen, plasmin, tPA or uPA. For example, "variants" of plasminogen, plasmin, tPA and uPA include mutational variants of these proteins obtained by substitution of such as 1-100, 1-90, 1-80, 1-70, 1-60, 1-50, 1- 45, 1-40, 1-35, 1-30, 1-25, 1-20, 1-15, 1-10, 1-5, 1-4, 1-3, 1-2, or 1 conservative amino acid.

A "plasminogen variant" of the application encompasses a protein comprising or having at least 75%, 80%, 85%, 90%, 95%, 96%, 97%, 98% or 99% sequence identity with an amino acid sequence of SEQ ID NO: 2, 6, 8, 10 or 12, and retaining plasminogen activity and/or lysine binding activity. For example, a "plasminogen variant" according to the present application may be a protein obtained by addition, deletion and/or substitution of 1-100, 1-90, 1-80, 1-70, 1-60, 1-50, 1-45, 1-40, 1-35, 1-30, 1-25, 1-20, 1-15, 1-10, 1-5, 1-4, 1-3, 1-2, or 1 amino acid on the basis of SEQ ID NO: 2, 6, 8, 10 or 12, and still retaining plasminogen activity and/or lysine binding activity. Particularly, the plasminogen variants according to the present application include all naturally occurring human genetic variants as well as other mammalian forms of these proteins, as well as mutational variants of these proteins obtained by substitution of such as 1-100, 1-90, 1-80, 1-70, 1-60, 1-50, 1-45, 1-40, 1-35, 1-30, 1-25, 1-20, 1-15, 1-10, 1-5, 1-4, 1-3, 1-2, or 1 conservative amino acid.

The plasminogen according to the present application may be a human plasminogen ortholog from a primate or rodent, or a variant thereof still retaining plasminogen activity and/or lysine binding activity, for example, a plasminogen represented by SEQ ID NO: 2, 6, 8, 10 or 12, such as a human natural plasminogen represented by SEQ ID NO: 2.

The "analogs" of the above plasminogen, plasmin, tPA, and uPA include compounds that respectively provide substantially similar effect to plasminogen, plasmin, tPA, or uPA.

The "variants" and "analogs" of above plasminogen, plasmin, tPA and uPA encompass "variants" and "analogs" of plasminogen, plasmin, tPA and uPA comprising one or more domains (e.g., one or more kringle domains and proteolytic domains). For example, "variants" and "analogs" of plasminogen encompass "variants" and "analogs" of plasminogen comprising one or more plasminogen domains (e.g., one or more kringle (k) domains and proteolytic domains, or called as serine protease domain, or plasminogen protease domain), such as mini-plasminogen. "Variants" and "analogs" of plasmin encompass "variants" and "analogs" of plasmin comprising one or more plasmin domains (e.g., one or more kringle domains and proteolytic domains), such as mini-plasmin, and delta-plasmin.

Whether a "variant" or "analog" of the above plasminogen, plasmin, tPA or uPA respectively has the activity of plasminogen, plasmin, tPA or uPA, or whether the "variant" or "analog" provides substantially similar effect to plasminogen, plasmin, tPA or uPA, may be detected by methods known in the art, for example, based on enzymography, ELISA (enzyme-linked immunosorbent assay), and FACS (fluorescence-activated cell sorting method), it is measured by the level of activated plasmin activity, for example, it is detected by referring to a method selected from the following documents: Ny, A., Leonardsson, G., Hagglund, A.C, Hagglof, P., Ploplis, V.A., Carmeliet, P. and Ny, T. (1999). Ovulation inplasminogen-deficient mice. Endocrinology 140, 5030-5035; Silverstein RL, Leung LL, Harpel PC, Nachman RL (November 1984). "Complex formation of platelet thrombospondin with plasminogen. Modulation of activation by tissue activator". J. Clin. Invest.74(5): 1625-33; Gravanis I, Tsirka SE (February 2008). "Tissue-type plasminogen activator as a therapeutic target in stroke". Expert Opinion on Therapeutic Targets. 12(2): 159-70; Geiger M, Huber K, Wojta J, Stingl L, Espana F, Griffin JH, Binder BR (Aug 1989). "Complex formation between urokinase and plasma protein C inhibitor in vitro and in vivo". Blood.74(2):722-8.

In some embodiments of the present application, the "component of plasminogen activation pathway" according to the present application is a plasminogen selected from the group consisting of: Glu-plasminogen, Lys-plasminogen, mini-plasminogen, micro-plasminogen, delta-plasminogen, or variants thereof retaining plasminogen activity. In some embodiments, the plasminogen is natural or synthetic human plasminogen, or a conservative mutant variant or fragment thereof still retaining plasminogen activity and/or lysine binding activity. In some embodiments, the plasminogen is a human plasminogen ortholog from a primate or rodent or a conservative mutant variant or fragment thereof still retaining plasminogen activity and/or lysine binding activity. In some embodiments, the amino acid sequence of the plasminogen comprises or has an amino acid sequence represented by SEQ ID NO: 2, 6, 8, 10 or 12. In some embodiments, the plasminogen is a human full length plasminogen. In some embodiments, the plasminogen is a human full length plasminogen represented by SEQ ID NO: 2.

"A compound capable of directly activating plasminogen, or indirectly activating plasminogen by activating a upstream component of plasminogen activation pathway", refers to any compound capable of directly activating plasminogen, or indirectly activating plasminogen by activating a upstream component of plasminogen activation pathway, such as tPA, uPA, streptokinase, saruplase, alteplase, reteplase, tenecteplase, anistreplase, monteplase, lanoteplase, pamiteplase, staphylokinase.

The "antagonist of a fibrinolysis inhibitor" according to the present application is a compound that antagonizes, weakens, blocks, or prevents the action of a fibrinolysis inhibitor. Such fibrinolysis inhibitors are e.g., PAI-1, complement C1 inhibitor, α2 antiplasmin, and α2 macroglobulin. Such an antagonist is: e.g., an antibody of PAI-1, complement C1 inhibitor, α2 antiplasmin, or α2 macroglobulin; or an antisense RNA or small RNA blocking or downregulating the expression of such as PAI-1, complement C1 inhibitor, α2 antiplasmin or α2 macroglobulin; or a compound occupying the binding site of PAI-1, complement C1 inhibitor, α2 antiplasmin, or α2 macroglobulin but without the function of PAI-1, complement C1 inhibitor, α2 antiplasmin, or α2 macroglobulin; or a compound blocking the binding and/or active domains of PAI-1, complement C1 inhibitor, α2 antiplasmin, or α2 macroglobulin.

Plasmin is a key component of the plasminogen activation system (PA system). It is a broad-spectrum protease capable of hydrolyzing several components of the extracellular matrix (ECM), including fibrin, gelatin, fibronectin, laminin, and proteoglycans. In addition, plasmin may activate some metalloproteinase precursors (pro-MMPs) to form active metalloproteinases (MMPs). Therefore, plasmin is considered to be an important upstream regulator of extracellular proteolysis. Plasmin is formed by proteolysis of plasminogen by two physiological PAs: tissue-type plasminogen activator (tPA) or urokinase-type plasminogen activator (uPA). Due to the relatively high levels of plasminogen in plasma and other body fluids, it has traditionally been thought that the regulation of the PA system is mainly achieved through the synthesis and activity levels of PAs. The synthesis of components of PA system is strictly regulated by different factors, such as hormone, growth factor and cytokine. In addition, there are specific physiological inhibitors of plasmin and PAs. The main inhibitor of plasmin is α2-antiplasmin. The activity of PAs is inhibited by plasminogen activator inhibitor-1 (PAI-1) of both uPA and tPA, and regulated by plasminogen activator inhibitor-2 (PAI-2) which mainly inhibits uPA. Certain cell surfaces have uPA-specific cell surface receptors (uPARs) with direct hydrolytic activity.

Plasminogen is a single-chain glycoprotein consisting of 791 amino acids with a molecular weight of approximately 92 kDa. Plasminogen is mainly synthesized in the liver, and is abundantly present in the extracellular fluid. The content of plasminogen in plasma is approximately 2 μM. Plasminogen is thus a huge potential source of proteolytic activity in tissues and body fluids. Plasminogen exists in two molecular forms: glutamate-plasminogen (Glu-plasminogen) and lysine-plasminogen (Lys-plasminogen). The naturally secreted and uncleaved form of plasminogen has an amino-terminal (N-terminal) glutamate, and is therefore referred to as glutamate-plasminogen. However, in the presence of plasmin, glutamate-plasminogen is hydrolyzed at Lys76-Lys77 into lysine-plasminogen. Compared with glutamate-plasminogen, lysine-plasminogen has a higher affinity for fibrin, and may be activated by PAs at a higher rate. The Arg560-Val561 peptide bond of these two forms of plasminogen may be cleaved by either uPA or tPA, resulting in the formation of two-chain protease plasmin linked by disulfide. The amino-terminal part of plasminogen comprises five homologous tri-cycles, i.e., so-called kringles, and the carboxy-terminal part comprises the protease domain. Some kringles comprise lysine-binding sites that mediate the specific interaction of plasminogen with fibrin and its inhibitor α2-AP. A recently found plasminogen is a 38 kDa fragment, including kringles1-4, and it is a potent inhibitor of angiogenesis. This fragment is named as angiostatin, and is produced by the hydrolysis of plasminogen by several proteases.

The main substrate of plasmin is fibrin, and the dissolution of fibrin is the key to preventing pathological thrombosis. Plasmin also has substrate specificity for several components of the ECM, including laminin, fibronectin, proteoglycans, and gelatin, indicating that plasmin also plays an important role in ECM remodeling. Indirectly, plasmin may also degrade other components of the ECM, including MMP-1, MMP-2, MMP-3 and MMP-9, by converting certain protease precursors into active proteases. Therefore, it has been proposed that plasmin may be an important upstream regulator of extracellular proteolysis. In addition, plasmin has the ability to activate certain latent forms of growth factors. In vitro, plasmin also hydrolyzes components of the complement system, and releases chemotactic complement fragments.

"Plasmin" is a very important enzyme present in the blood that hydrolyzes fibrin clots into fibrin degradation products and D-dimers.

"Plasminogen" is the zymogen form of plasmin. According to the sequence in swiss prot, it consists of 810 amino acids calculating by the natural human plasminogen amino acid sequence (SEQ ID NO: 4) containing the signal peptide, and the molecular weight is about 90kD, and it is a glycoprotein mainly synthesized in the liver and capable of circulating in the blood, the cDNA sequence encoding this amino acid sequence is represented by SEQ ID NO: 3. Full-length plasminogen contains seven domains: a C-terminal serine protease domain, an N-terminal Pan Apple (PAp) domain, and five Kringle domains (Kringle1-5). Referring to the sequence in swiss prot, its signal peptide comprises residues Met1-Gly19, PAp comprises residues Glu20-Val98, Kringle1 comprises residues Cys103-Cys181, Kringle2 comprises residues Glu184-Cys262, Kringle3 comprises residues Cys275-Cys352, Kringle4 comprises residues Cys377-Cys454, and Kringle5 comprises residues Cys481-Cys560. According to NCBI data, the serine protease domain comprises residues Val581-Arg804.

Glu-plasminogen is a natural full-length plasminogen, consisting of 791 amino acids (without a signal peptide of 19 amino acids); the cDNA sequence encoding this amino acid sequence is represented by SEQ ID NO: 1, and the amino acid sequence is represented by SEQ ID NO: 2. In vivo, there is also a Lys-plasminogen produced by the hydrolysis of the peptide bond between amino acids 76 and 77 of Glu-plasminogen, as represented by SEQ ID NO: 6; and the cDNA sequence encoding this amino acid sequence is represented by SEQ ID NO: 5. Delta-plasminogen (δ-plasminogen) is a fragment of full-length plasminogen that lacks the Kringle2-Kringle5 structure, and only contains Kringle1 and a serine protease domain (also known as a proteolysis domain, or a plasminogen protease domain). The amino acid sequence of delta-plasminogen (SEQ ID NO: 8) is reported in a literature, and the cDNA sequence encoding this amino acid sequence is represented by SEQ ID NO: 7. Mini-plasminogen consists of Kringle5 and a serine protease domain, and it is reported that it comprises residues Val443-Asn791 (with the Glu residue of the Glu-plasminogen sequence without the signal peptide as the starting amino acid), its amino acid sequence is represented by SEQ ID NO: 10, and the cDNA sequence encoding this amino acid sequence is represented by SEQ ID NO: 9. While micro-plasminogen comprises only a serine protease domain, and it is reported that its amino acid sequence comprises residues Ala543-Asn791 (with the Glu residue of the Glu-plasminogen sequence without the signal peptide as the starting amino acid); additionally, it is disclosed in patent document CN102154253A that its sequence comprises residues Lys531-Asn791 (with the Glu residue of the Glu-plasminogen sequence without the signal peptide as the starting amino acid); the sequence of the present patent application refers to the patent document CN102154253A, the amino acid sequence is represented by SEQ ID NO: 12, and the cDNA sequence encoding this amino acid sequence is represented by SEQ ID NO: 11.

In the present application, "plasmin" and "fibrinolytic enzyme" may be used interchangeably with the same meaning; "plasminogen" and "fibrinolytic zymogen" may be used interchangeably with the same meaning.

In the present application, "lack" of plasminogen or plasminogen activity means that the content of plasminogen in a subject is lower than that of a normal person, and is sufficiently low to affect the normal physiological function of the subject; "deficiency" of plasminogen or plasminogen activity means that the content of plasminogen in a subject is significantly lower than that of a normal person, and even the activity or expression is extremely low, and the normal physiological function may only be maintained by external supply of plasminogen.

Those skilled in the art may understand that, all technical solutions of plasminogen according to the present application are applicable to plasmin, thus the technical solutions described in the present application encompass plasminogen and plasmin. During circulation, plasminogen is present in a closed, inactive conformation, but when bound to a thrombus or cell surface, it is converted into active plasmin with an open conformation after being mediated by plasminogen activator (PA). Active plasmin may further hydrolyze the fibrin clot into degradation products of fibrin and D-dimers, thereby dissolving the thrombus. The PAp domain of plasminogen comprises an important determinant for maintaining plasminogen in an inactive closed conformation, while the KR domain may bind to a lysine residue present on a receptor and substrate. A variety of enzymes are known to act as plasminogen activators, including: tissue plasminogen activator (tPA), urokinase plasminogen activator (uPA), kallikrein, and coagulation factor XII (Hageman factor) etc.

"Active fragments of plasminogen" in this application include 1) in plasminogen protein, active fragments capable of binding to target sequences in substrates, also known as lysine-binding fragments, for example fragments of comprising Kringle1, Kringle 2, Kringle 3, Kringle 4 and/or Kringle 5 (for the structure of the plasminogen, see Aisina RB, Mukhametova L I. Structure and function of plasminogen/plasmin system [J]. Russian Journal of Bioorganic Chemistry, 2014,40(6):590-605); 2) an active fragment that plays a proteolytic function in the plasminogen protein, e.g., a fragment comprising the plasminogen activity (proteolytic function) represented by SEQ ID NO: 14; 3) in the plasminogen protein, a fragment that has both the binding activity to the target sequence in the substrate (lysine binding activity) and the plasminogen activity (proteolytic function). In some embodiments of the present application, the plasminogen is a protein comprising the active fragment of plasminogen represented by SEQ ID NO: 14. In some embodiments of the present application, the plasminogen is a protein comprising a lysine-binding fragment of Kringle 1, Kringle 2, Kringle 3, Kringle 4 and/or Kringle 5. In some embodiments, the plasminogen active fragment of the present application comprises SEQ ID NO: 14, a protein with an amino acid sequence having at least 80%, 90%, 95%, 96%, 97%, 98%, 99% homology with SEQ ID NO: 14. Therefore, the plasminogen of the present application comprises a protein comprising the active fragment of the plasminogen and still maintaining the activity of the plasminogen. In some embodiments, the plasminogen of the present application comprises Kringle 1, Kringle 2, Kringle 3, Kringle 4 and/or Kringle 5, or a protein has at least 80%, 90%, 95%, 96%, 97%, 98%, 99% homologous with Kringle 1, Kringle 2, Kringle 3, Kringle 4 and/or Kringle 5 and still have lysine-binding activity.

At present, the methods for measuring plasminogen and its activity in blood comprise:
detection of tissue plasminogen activator activity (t-PAA), detection of plasma tissue plasminogen activator antigen (t-PAAg), detection of plasma tissue plasminogen activity (plgA), detection of plasma tissue plasminogen antigen (plgAg), detection of the activity of plasma tissue plasminogen activator inhibitor, detection of the antigen of plasma tissue plasminogen activator inhibitor, and detection of plasma plasmin-antiplasmin complex (PAP); wherein the most commonly used detection method is the chromogenic substrate method: adding streptokinase (SK) and a chromogenic substrate to the plasma to be detected, the PLG in the plasma to be detected is converted into PLM under the action of SK, and PLM acts on the chromogenic substrate; subsequently, the detection by spectrophotometer indicates that the increase in absorbance is proportional to plasminogen activity. In addition, the plasminogen activity in blood may also be detected by immunochemical method, gel electrophoresis, immunoturbidimetry, and radioimmunoassay.

"Ortholog or orthologs" refer to homologs between different species, including both protein homologs and DNA homologs, also known as orthologs and vertical homologs; particularly it refers to proteins or genes evolved from the same ancestral gene in different species. The plasminogen according to the present application includes human natural plasminogen, and also includes plasminogen ortholog or orthologs derived from different species and having plasminogen activity.

A "conservative substitution variant" refers to a variant in which a given amino acid residue is altered without changing the overall conformation and function of the protein or enzyme, including but not limited to those variants in which the amino acid(s) in the amino acid sequence of the parent protein are replaced by amino acid(s) with similar properties (e.g., acidic, basic, hydrophobic, etc.). Amino acids with similar properties are well known in the art. For example, arginine, histidine and lysine are hydrophilic basic amino acids and are interchangeable. Similarly, isoleucine is a hydrophobic amino acid, and may be replaced by leucine, methionine or valine. Therefore, the similarity of two proteins or amino acid sequences with similar functions may differ; for example, 70% to 99% similarity (identity) based on the MEGALIGN algorithm. "Conservative substitution variants" also include polypeptides or enzymes having more than 60%, preferably more than 75%, more preferably more than 85%, or even most preferably more than 90% amino acid identity determined by BLAST or FASTA algorithm, and having the same or substantially similar properties or functions as the natural or parent protein or enzyme.

"Isolated" plasminogen refers to a plasminogen protein isolated and/or recovered from its natural environment. In some embodiments, the plasminogen will be purified: (1) to more than 90%, more than 95%, or more than 98% purity (by weight), as determined by Lowry's method, e.g., more than 99% (by weight), (2) to a degree sufficient to obtain at least 15 residues of the N-terminal or internal amino acid sequence by using a spinning cup sequence analyzer, or (3) to homogeneity as determined by using Coomassie blue or silver staining through sodium dodecyl sulfate-polyacrylamide gel electrophoresis (SDS-PAGE) under reducing or non-reducing conditions. Isolated plasminogen also includes plasminogen prepared from recombinant cells by bioengineering techniques and isolated by at least one purification step.

The terms "polypeptide", "peptide" and "protein" are used interchangeably herein to refer to a polymeric form of amino acids of any length, which may include genetically encoded and non-genetically encoded amino acids, chemically or biochemically modified or derivatized amino acids, and polypeptides with modified peptide backbones. The terms include fusion proteins including, but not limited to, fusion proteins with heterologous amino acid sequences, fusions with heterologous and homologous leader sequences (with or without N-terminal methionine residues); and the like.

"Percent (%) of amino acid sequence identity" with respect to a reference polypeptide sequence is defined as, after introducing gaps as necessary to achieve maximum percent sequence identity, and no conservative substitutions are considered as part of the sequence identity, the percentage of amino acid residues in a candidate sequence that are identical to the amino acid residues in a reference polypeptide sequence. Alignment for purposes of determining percent amino acid sequence identity may be accomplished in a variety of ways within the technical scope in the art, e.g., by publicly available computer software, such as BLAST, BLAST-2, ALIGN or Megalign (DNASTAR) software. Those skilled in the art may determine the appropriate parameters for aligning sequences, including any algorithms needed to achieve maximal alignment over the full length of the sequences to be compared. However, for the purpose of the present application, the values of percent amino acid sequence identity are generated by using the computer program ALIGN-2 for sequence comparison.

Where ALIGN-2 is used to compare amino acid sequences, the percentage (%) of amino acid sequence identity of a given amino acid sequence A relative to a given amino acid sequence B (or may be expressed as a given amino acid sequence A having a certain percentage (%) of amino acid sequence identity relative to, with or with respective to a given amino acid sequence B) is calculated as follows:

$$\text{Fraction } X/Y \text{ times } 100;$$

wherein X is the number of amino acid residues scored as identical matches during the alignment of sequences A and B by the sequence alignment program ALIGN-2, and wherein Y is the total number of amino acid residues in sequence B. It should be appreciated that, where the length of amino acid sequence A is not equal to that of amino acid sequence B, the percentage (%) of amino acid sequence identity of A with respect to B will not equal to the percentage (%) of amino acid sequence identity of B with respect to A. Unless expressly stated otherwise, all the values of percentage (%) of amino acid sequence identity used herein are obtained by using the ALIGN-2 computer program as described in the preceding paragraph.

The terms "individual", "subject" and "patient" are used interchangeably herein to refer to mammals including, but not limited to, murine (rat, mouse), non-human primate, human, canine, feline, hoofed animals (e.g., horses, cattle, sheep, pigs, goats), etc.

A "therapeutically effective amount" or "effective amount" refers to an amount of plasminogen sufficient to prevent and/or treat a disease when administrated to a mammal or other subject for treating the disease. A "therapeutically effective amount" will vary depending on the plasminogen used, the severity of the disease and/or symptoms thereof in the subject to be treated, as well as the age, weight, and the like.

The term "treatment" of a disease state includes inhibiting or preventing the development of the disease state or its clinical symptoms, or alleviating the disease state or symptoms, resulting in temporary or permanent regression of the disease state or its clinical symptoms.

**Preparation of the Plasminogen According to the Present Application**

Plasminogen may be isolated from nature, and purified for further therapeutic use, or it may be synthesized by standard chemical peptide synthesis techniques. When the polypeptide is synthesized chemically, the synthesis may be carried out via liquid phase or solid phase. Solid-phase polypeptide synthesis (SPPS) (in which the C-terminal amino acid of the sequence is attached to an insoluble support, followed by the sequential addition of the retaining amino acids in the sequence) is a suitable method for chemical synthesis of plasminogen. Various forms of SPPS, such as Fmoc and Boc, may be used to synthesize plasminogen. Techniques for solid-phase synthesis are described in Barany and Solid-Phase Peptide Synthesis; pp.3-284 in The Peptides: Analysis, Synthesis, Biology. Vol. 2: Special Methods in Peptide Synthesis, Part A., Merrifield, et al. J. Am. Chem. Soc., 85:2149-2156 (1963); Stewart et al., Solid Phase Peptide Synthesis, 2nd ed. Pierce Chem. Co., Rockford, Ill. (1984); and Ganesan A. 2006 Mini Rev. Med Chem. 6:3-10, and Camarero JA et al. 2005, Protein Pept Lett. 12:723-8. Briefly, small insoluble porous beads are treated with functional units on which peptide chains are constructed; after repeated cycles of coupling/deprotection, the attached solid-phase free N-terminal amine is coupled to a single N-protected amino acid unit. This unit is then deprotected to reveal new N-terminal amines that may be attached to other amino acids. The peptide remains immobilized on the solid phase, subsequently it is cleaved off.

Plasminogen according to the present application may be produced by standard recombinant methods. For example, a nucleic acid encoding plasminogen is inserted into an expression vector to operably link to regulatory sequences in the expression vector. The regulatory sequences for expression include, but are not limited to, promoters (e.g., naturally associated or heterologous promoters), signal sequences, enhancer elements, and transcription termination sequences. Expression regulation may be a eukaryotic promoter system in a vector capable of transforming or transfecting a eukaryotic host cell (e.g., COS or CHO cell). Once the vector is incorporated into a suitable host, the host is maintained under conditions suitable for high-level expression of the nucleotide sequence and collection and purification of plasminogen.

A suitable expression vector typically replicates in a host organism as an episome or as an incorporated part of the host chromosomal DNA. Typically, an expression vector contains a selectable marker (e.g., ampicillin resistance, hygromycin resistance, tetracycline resistance, kanamycin resistance, or neomycin resistance marker) to facilitate the detection of those cells transformed with desired exogenous DNA sequence.

*Escherichia coli* is an example of a prokaryotic host cell that may be used to clone a plasminogen-encoding polynucleotide. Other microbial hosts suitable for use include bacilli such as *Bacillus subtilis,* and other *enterobacteriaceae* such as *Salmonella, Serratia,* and various *Pseudomonas* species. In these prokaryotic hosts, expression vectors may also be generated, which will typically contain an expression control sequence (e.g., origin of replication) that are compatible with the host cell. In addition, there are many well-known promoters, such as the lactose promoter system, the tryptophan (trp) promoter system, the beta-lactamase promoter system, or the promoter system from bacteriophage lambda. A promoter will typically control the expression, optionally in case of an operator gene sequence, and have ribosome binding site sequence, etc., to initiate and complete transcription and translation.

Other microorganisms, such as yeast, may also be used for expression. Yeast (e.g., *S. cerevisiae*) and *Pichia* are examples of suitable yeast host cells, and as required a suitable vector has an expression control sequence (e.g., promoter), origin of replication, termination sequence, etc. A typical promoter comprises 3-phosphoglycerate kinase and other saccharolytic enzymes. Particularly, inducible yeast promoters include promoters from ethanol dehydrogenase, isocytochrome C, and enzymes responsible for maltose and galactose utilization

In addition to microorganisms, mammalian cells (e.g., mammalian cells grown in *in vitro* cell culture) may also be used to express and produce the plasminogen of the application (e.g., polynucleotides encoding the plasminogen). See Winnacker, From Genes to Clones, VCH Publishers, N.Y, N.Y. (1987). Suitable mammalian host cells include CHO cell lines, various Cos cell lines, HeLa cells, myeloma cell lines, and transformed B cells or hybridomas. Expression vectors for use in these cells may comprise expression control sequences such as origin of replication, promoter and enhancer (Queen et al., Immunol. Rev. 89:49 (1986)), and necessary sites for processing information such as ribosome binding sites, RNA splicing sites, polyadenylation sites, and transcription terminator sequences. Examples of suitable expression control sequences are promoters derived from immunoglobulin gene, SV40, adenovirus, bovine papilloma virus, cytomegalovirus, and the like. See Co et al, J. Immunol. 148:1149 (1992).

Once synthesized (chemically or recombinantly), the plasminogen of the present application may be purified according to standard procedures in the art, including ammonium sulfate precipitation, affinity column, column chromatography, high performance liquid chromatography (HPLC), gel electrophoresis, and the like. The plasminogen is substantially pure, e.g., at least about 80-85% pure, at least about 85-90% pure, at least about 90-95% pure, or 98-99% pure or purer, e.g., free of contaminants such as cellular debris, macromolecules other than the plasminogen, and the like.

**Medicament Formulation**

A therapeutic formulation may be prepared by mixing the plasminogen of desired purity with an optional pharmaceutical carrier, excipient, or stabilizer (Remington's Pharmaceutical Sciences, 16th edition, Osol, A. ed. (1980)), to form a lyophilized formulation or aqueous solution. An acceptable carrier, excipient, or stabilizer is non-toxic to a recipient at the employed dosage and concentration, including buffers such as phosphate, citrate and other organic acids; antioxidants such as ascorbic acid and methionine; preservatives (such as octadecyldimethylbenzylammonium chloride; hexanediamine chloride; benzalkonium chloride, benzethonium chloride; phenol, butanol or benzyl alcohol; alkyl parahydroxybenzoate such as methyl or propyl paraben; catechol; resorcinol; cyclohexanol; 3-pentanol; m-cresol); low molecular weight polypeptides (less than about 10 residues); proteins such as serum albumin, gelatin or immunoglobulins; hydrophilic polymers such as polyvinylpyrrolidone; amino acids such as glycine, glutamine, asparagine, histidine, arginine or lysine; monosaccharides, disaccharides and other carbohydrates such as glucose, mannose, or dextrin; chelating agents such as EDTA; carbohydrates such as sucrose, mannitol, fucose, or sorbitol; salt-forming counterions such as sodium; metal complexes (such as zinc-protein complexes); and/or nonionic surfactants such as TWENTM, PLURONICSTM or polyethylene glycol (PEG).

The formulations according to the present application may also contain more than one active compound as required for the particular condition to be treated, preferably those compounds are complementary in activity and do not have side effects with each other. For example, antihypertensive medicaments, antiarrhythmic medicaments, medicaments for treating diabetes, etc.

The plasminogen according to the present application may be encapsulated in microcapsules prepared by techniques such as coacervation or interfacial polymerization, for example, the plasminogen may be placed in colloidal drug delivery systems (e.g., liposomes, albumin microspheres, microemulsions, nanoparticles and nanocapsules) or in hydroxymethyl cellulose or gel-microcapsules and poly-(methyl methacrylate) microcapsules in macroemulsions. These techniques are disclosed in Remington's Pharmaceutical Sciences 16th edition, Osol, A. Ed. (1980).

The plasminogen according to the present application for *in vivo* administration must be sterile. This may be easily achieved by filtration through sterilizing filters before or after lyophilization and reformulation.

The plasminogen according to the present application may be prepared as a sustained-release formulation. Suitable examples of sustained-release formulations include semipermeable matrices of solid hydrophobic polymers which have a certain shape and contain glycoprotein, for example, membranes or microcapsules. Examples of sustained-release matrices include polyesters, hydrogels such as poly(2-hydroxyethyl-methacrylate) (Langer et al., J. Biomed. Mater. Res., 15:167-277 (1981); Langer, Chem. Tech., 12:98-105 (1982)), or poly(vinyl alcohol), polylactide (US Pat. No.3,773,919, EP58,481), copolymers of L-glutamic acid and $\gamma$-ethyl-L-glutamic acid (Sidman, et al., Biopolymers 22:547 (1983)), non-degradable ethylene-vinyl acetate (Langer, et al., supra), or degradable lactic acid-glycolic acid copolymers such as Lupron Depot™ (injectable microspheres consisting of lactic acid-glycolic acid copolymer and leuprolide acetate), and poly-D-(-)-3-hydroxybutyric acid. Polymers such as ethylene-vinyl acetate and lactic acid-glycolic acid may release molecules continuously for more than 100 days, while some hydrogels release proteins for shorter period of time. Rational strategies to stabilize proteins may be devised based on the relevant mechanisms. For example, if the mechanism of condensation is found to form intermolecular S-S bond through thiodisulfide interchange, then stabilization may be achieved by modifying sulfhydryl residues, lyophilizing from acidic solutions, controlling humidity, using suitable additives, and developing specific polymer matrix composition.

**Administration and Dosage**

Administration of the pharmaceutical composition according to the present application may be accomplished by different means, e.g., intravenously, intraperitoneally, subcutaneously, intracranially, intrathecally, intraarterally (e.g., via the carotid artery), and intramuscularly.

Preparations for parenteral administration include sterile aqueous or non-aqueous solutions, suspensions and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, or fixed oils. Intravenous vehicles include fluid and nutritional supplements, electrolyte supplements, and the like. Preservatives and other additives may also be present, such as, for example, antimicrobials, antioxidants, chelating agents, and inert gases, etc.

Dosing regimens will be determined by medical personnel based on various clinical factors. As is well known in the medical field, the dosage for any patient depends on a variety of factors, including the patient's size, body surface area, age, the particular compound to be administrated, sex, number and route of administration, general health, and other concomitantly administrated medicaments. The dosage range of the pharmaceutical composition comprising the plasminogen according to the present application may be about 0.0001-2000 mg/kg, or about 0.001-500 mg/kg (e.g., 0.02 mg/kg, 0.25 mg/kg, 0.5 mg/kg, 0.75 mg/kg, 10 mg/kg, 50 mg/kg, etc.) body weight of the subject per day. For example, the dose may be 1 mg/kg body weight, or 50 mg/kg body weight, or in the range of 1-50 mg/kg, or at least 1 mg/kg. Dosages above or below this exemplary range are also contemplated, especially in view of the factors set forth above. Intermediate doses within the above ranges are also included within the scope of the present application. Subjects may be administrated such doses daily, every other day, weekly, or according to any other schedule determined by empirical analysis. An exemplary dosage schedule includes 1-10 mg/kg on consecutive days. Real-time evaluation of therapeutic efficacy and safety is required during the administration of the medicament of the present application.

**Product or Kit**

One embodiment of the present application relates to a product or kit comprising the plasminogen or plasmin according to the present application for treating cardiovascular disease caused by diabetes and related disorders. The product preferably comprises a container, and a label or package insert. Suitable containers are bottles, vials, syringes, etc. The container may be made of various materials such as glass or plastic. The container contains a composition which is effective for treatment of the disease or disorder according to the present application and has a sterile access port (e.g., the container may be an intravenous solution pack or vial containing a stopper penetrable by a hypodermic needle). At least one active agent in the composition is plasminogen/plasmin. The label on or attached to the container indicates that the composition is used for treatment of cardiovascular disease caused by diabetes and related disorders according to the present application. The product may further comprise a second container containing a pharmaceutically acceptable buffer, such as phosphate buffered saline, Ringer's solution, and dextrose solution. It may further contain other materials required from a commercial and user standpoint, including other buffers, diluents, filters, needles and syringes. In addition, the product comprises a package insert with instructions for use, including, for example, instructing the user of the composition to administrate the plasminogen composition to the patient along with other medicaments for treatment of concomitant diseases.

EXAMPLES

Human plasminogen used in all the following examples is derived from plasma of a donor. The process is optimized based on methods described in the References 1-3, and the human plasminogen is purified from human donor plasma, wherein human Lys-plasminogen and Glu-plasminogen >98%.

**Example 1: Plasminogen Promotes the Cleavage of Pro-BDNF in Brain Homogenate of Normal Mice**

Four C57BL/6J male mice of 11-12 week-old with weight of 18-25 g were sacrificed to take out the whole brain and weigh, then adding 1×PBS (Thermo Fisher, pH7.4; 10010-031) respectively at 150mg tissue/mL PBS to homogenize at 4°C (Imin, 3-4 times), after homogenization, centrifuging at 4°C (12000rpm, 20min), taking the supernatant (i.e., the brain homogenate) to place it in a new EP tube.

Eppendorf (EP) tubes were taken to set them as: ①blank group, ②blank control group, ③ vehicle control group, and ④plasminogen group, with 5 parallel sets in each group. Particularly, 21.5 µL of normal saline, 4.6 µL of plasminogen solution (2mg/mL), and 23.9 µL of mouse brain homogenate were added in the blank control group; 21.5 µL of Pro-BDNF (Shanghai QyaoBio, custom-expressed human Pro-BDNF, 1.0mg/mL), 4.6µL of vehicle solution (citric acid-sodium citrate solution), 23.9µL of mouse brain homogenate were added in the vehicle control group; 21.µL of Pro-BDNF (1.0mg/mL), 4.6µL of plasminogen solution (2mg/mL), 23.9µL of mouse brain homogenate were added in the plasminogen group. After the samples were added in each group, incubating at 37°C for 6 h, then 100 µL of 0.1% trifluoroacetic acid solution was added to terminate the reaction.

A 12% gel was prepared according to the SDS-PAGE gel formulation instructions. Samples in each group was respectively mixed well with 4× loading buffer (TaKaRa, e2139) at a volume ratio of 3:1, then heating at 100°C for 5 min, cooling to centrifuge for 2 min, and then 20 µL was taken for loading. The electrophoresis condition is 30V to run for 30min, then 100V electrophoresis to the bottom of the gel. After electrophoresis, the gel was stripped and stained with 1‰ Coomassie Brilliant Blue staining solution (1g Coomassie Brilliant Blue R250 dissolved in 1000ml mixture of ethanol: glacial acetic acid: purified water at a volume ratio of 5:2:13) for 30min, then decolorizing until clean by decolorization solution (a mixture of purified water: glacial acetic acid: absolute ethanol = a volume ratio of 17:2:1). The gel was photographed under a biomolecular imager, and quantitatively scanned for analysis.

BDNF is a kind of basic protein with a molecular weight of 12.3kD, consisting of 119 amino acid residues and containing 3 pairs of disulfide bonds. It exists in the form of dimers *in vivo,* and is synthesized in the form of BDNF precursor. BDNF precursor (Pro-BDNF) can be cleaved by enzymolysis to form mature BDNF. It has been reported in the literature that, Pro-BDNF has the opposite effect as compared with the mature BDNF formed by its cleavage. Pro-BDNF promotes apoptosis of nerve cells, and reduces synaptic plasticity [1]. Mature BDNF and its receptors are widely distributed in the central nervous system. During the development of the central nervous system, they play an important role in the survival, differentiation, growth and development of neurons, and can prevent neurons from being injured and dying, improve the pathological state of neurons, promote biological effects such as regeneration and differentiation of injured neurons, and they are also necessary for the survival and normal physiological functions of mature neurons in the central and peripheral nervous systems [2].

The results show that in brain homogenate of normal mice, the amount of Pro-BDNF in the plasminogen group is significantly lower than that in the vehicle control group, and the difference is extremely significant (*** represents P<0.001) (Fig. 1). It is suggested that plasminogen can promote the cleavage of Pro-BDNF in brain homogenate of normal mice.

**Example 2: Plasminogen Promotes the Cleavage of Pro-BDNF in Brain Homogenate of Normal Mice to Form Mature BDNF**

Four C57BL/6J male mice of 11-12 week-old with weight of 18-25 g were sacrificed to take out the whole brain and weigh, then adding 1×PBS (Thermo Fisher, pH7.4; 10010-031) respectively at 150mg tissue/mL PBS to homogenize at 4°C (Imin, 3-4 times), after homogenization, centrifuging at 4°C (12000rpm, 20min), taking the supernatant (i.e., the brain homogenate) to place it in a new EP tube.

Eppendorf (EP) tubes were taken to set them as: ①blank group, ②blank control group, ③ vehicle control group, and ④plasminogen group, with 5 parallel sets in each group. Particularly, 21.5 μL of normal saline, 4.6 μL of plasminogen solution (2mg/mL), and 23.9 μL of mouse brain homogenate were added in the blank control group; 21.5 μL of Pro-BDNF (Shanghai QyaoBio, custom-expressed human Pro-BDNF, 1.0mg/mL), 4.6μL of vehicle solution (citric acid-sodium citrate solution), 23.9μL of mouse brain homogenate were added in the vehicle control group; 21.μL of Pro-BDNF (1.0mg/mL), 4.6μL of plasminogen solution (2mg/mL), 23.9μL of mouse brain homogenate were added in the plasminogen group. After the samples were added in each group, incubating at 37°C for 6 h, then 100 μL of 0.1% trifluoroacetic acid solution was added to terminate the reaction.

A 10% gel was prepared according to the SDS-PAGE gel formulation instructions. Samples in each group was respectively mixed well with 4× loading buffer (TaKaRa, e2139) at a volume ratio of 3:1, then heating at 100°C for 5 min, cooling to centrifuge for 2 min, and then 20 μL was taken for loading. The electrophoresis condition is 30V to run for 45min, then 100V electrophoresis to the bottom of the gel. After electrophoresis, the gel was stripped and transferred to an activated PVDF membrane (GE, A29433753), and the electrophoresis condition was 15V for 2.5h. The transferred PVDF membrane was soaked in blocking solution (5% skim emulsion) to block overnight in a refrigerator at 4°C, washing 4 times with TBST (0.01M Tris-NaCl, pH7.6 buffer), then adding rabbit anti-human BDNF antibody (Boster Biological Technology, PB9075) to incubate at room temperature for 3 h, washing 4 times with TBST, adding goat anti-rabbit IgG (HRP) antibody (Abcam, ab6721) secondary antibody to incubate at room temperature for 1 h, washing 4 times with TBST, placing the PVDF membrane on a clean imaging plate, then adding Immobilon Western HRP Substrate (MILLIPORE, WBKLS0100) for color development, photographing under a biomolecular imager, and performing quantitative analysis with Image J.

The results show that in brain homogenate of normal mice, the amount of Pro-BDNF in the plasminogen group is significantly lower than that in the vehicle control group, and the difference is extremely significant (** represents P<0.01) (Fig. 2). It is suggested that plasminogen can promote the cleavage of Pro-BDNF in brain homogenate of normal mice.

**Example 3: Plasminogen Promotes Cleavage of Pro-BDNF in Brain Homogenate of Parkinson's Disease Model Mice**

Four C57BL/6J male mice of 11-12 week-old with weight of 18-25 g were taken. The modeling time was set at 9:00 every morning, particularly the blank control group was intraperitoneally injected with 200 μL of normal saline; the model group was intraperitoneally injected with 5 mg/mL MPTP solution at 35 mg/kg/mouse for 5 consecutive days to establish a Parkinson's disease model[5]. Preparation of MPTP solution: 45 mg of MPTP (sigma, M0896) was dissolved in 9 mL of normal saline solution, and the final concentration of the preparation was 5 mg/mL. After the modeling was completed, i.e., on day 6 after the modeling, all mice were subjected to an open-field test to confirm that the modeling was successful. After all the mice were sacrificed, the whole brain was taken and weighed, and 1×PBS (Thermo Fisher, pH 7.4; 10010-031) was respectively added at 150 mg tissue/mL PBS to homogenize at 4°C (1 min, 3-4 times). After homogenization, the mixture was centrifuged at 4°C (12000rpm, 20min), and the supernatant (i.e., the brain homogenate) was taken to place in a new EP tube.

Eppendorf (EP) tubes were taken to set them as: ①blank group, ②blank control group, ③ vehicle control group, and ④plasminogen group, with 5 parallel sets in each group. Particularly, 21.5 μL of normal saline, 4.6 μL of plasminogen solution (2mg/mL), and 23.9 μL of mouse brain homogenate were added in the blank control group; 21.5 μL of Pro-BDNF (Shanghai QyaoBio, custom-expressed human Pro-BDNF, 1.0mg/mL), 4.6μL of vehicle solution (citric acid-sodium citrate solution), 23.9μL of mouse brain homogenate were added in the vehicle control group; 21.μL of Pro-BDNF (1.0mg/mL), 4.6μL of plasminogen solution (2mg/mL), 23.9μL of mouse brain homogenate were added in the plasminogen group. After the samples were added in each group, incubating at 37°C for 6 h, then 100 μL of 0.1% trifluoroacetic acid solution was added to terminate the reaction.

A 12% gel was prepared according to the SDS-PAGE gel formulation instructions. Samples in each group was respectively mixed well with 4× loading buffer (TaKaRa, e2139) at a volume ratio of 3:1, then heating at 100°C for 5 min, cooling to centrifuge for 2 min, and then 20 μL was taken for loading. The electrophoresis condition is 30V to run for 45min, then 100V electrophoresis to the bottom of the gel. After electrophoresis, the gel was stripped and stained with 1‰ Coomassie Brilliant Blue staining solution (1g Coomassie Brilliant Blue R250 dissolved in 1000ml mixture of ethanol: glacial acetic acid: purified water at a volume ratio of 5:2:13) for 30min, then decolorizing until clean by decolorization solution (a mixture of purified water: glacial acetic acid: absolute ethanol = a volume ratio of 17:2:1). The gel was photographed under a biomolecular imager, and quantitatively scanned for analysis.

The results show that in the brain homogenate of Parkinson's disease model mice, the amount of Pro-BDNF in the plasminogen group is significantly lower than that in the vehicle control group, and the difference is extremely significant (*** represents P<0.001) (Fig. 3). It is suggested that plasminogen can promote the cleavage of Pro-BDNF in brain homogenate of Parkinson's disease model mice.

**Example 4: Plasminogen Promotes Cleavage of Pro-BDNF in the Brain Homogenate of Parkinson's Disease Model Mice to Form Mature BDNF**

Eight C57BL/6J male mice of 11-12 week-old with weight of 18-25 g were taken to weigh one day before modeling, and randomly divided into 2 groups according to body weight, particularly 4 mice in the blank control group, and 4 mice in the model group. The modeling time was set at 9:00 every morning, particularly the blank control group was intraperitoneally injected with 200 $\mu$L of normal saline; the model group was intraperitoneally injected with 5 mg/mL MPTP solution at 35 mg/kg/mouse for 5 consecutive days to establish a Parkinson's disease model[5]. Preparation of MPTP solution: 45 mg of MPTP (sigma, M0896) was dissolved in 9 mL of normal saline solution, and the final concentration of the preparation was 5 mg/mL. After the modeling was completed, i.e., on day 6 after the modeling, all mice were subjected to an open-field test to confirm that the modeling was successful. After all the mice were sacrificed, the whole brain was taken and weighed, and 1$\times$PBS (Thermo Fisher, pH 7.4; 10010-031) was respectively added at 150 mg tissue/mL PBS to homogenize at 4°C (1 min, 3-4 times). After homogenization, the mixture was centrifuged at 4°C (12000rpm, 20min), and the supernatant (i.e., the brain homogenate) was taken to place in a new EP tube.

Eppendorf (EP) tubes were taken to set them as: ①blank group, ②blank control group, ③ vehicle control group, and ④plasminogen group, with 5 parallel sets in each group. Particularly, 21.5 $\mu$L of normal saline, 4.6 $\mu$L of plasminogen solution (2mg/mL), and 23.9 $\mu$L of mouse brain homogenate were added in the blank control group; 21.5 $\mu$L of Pro-BDNF (Shanghai QyaoBio, custom-expressed human Pro-BDNF, 1.0mg/mL), 4.6$\mu$L of vehicle solution (citric acid-sodium citrate solution), 23.9$\mu$L of mouse brain homogenate were added in the vehicle control group; 21.$\mu$L of Pro-BDNF (1.0mg/mL), 4.6$\mu$L of plasminogen solution (2mg/mL), 23.9$\mu$L of mouse brain homogenate were added in the plasminogen group. After the samples were added in each group, incubating at 37°C for 6 h, then 100 $\mu$L of 0.1% trifluoroacetic acid solution was added to terminate the reaction.

A 10% gel was prepared according to the SDS-PAGE gel formulation instructions. Samples in each group was respectively mixed well with 4$\times$ loading buffer (TaKaRa, e2139) at a volume ratio of 3:1, then heating at 100°C for 5 min, cooling to centrifuge for 2 min, and then 20 $\mu$L was taken for loading. The electrophoresis condition is 30V to run for 45min, then 100V electrophoresis to the bottom of the gel. After electrophoresis, the gel was stripped and transferred to an activated PVDF membrane (GE, A29433753), and the electrophoresis condition was 15V for 2.5h. The transferred PVDF membrane was soaked in blocking solution (5% skim emulsion) to block overnight in a refrigerator at 4°C, washing 4 times with TBST (0.01M Tris-NaCl, pH7.6 buffer), then adding rabbit anti-human BDNF antibody (Boster Biological Technology, PB9075) to incubate at room temperature for 3 h, washing 4 times with TBST, adding goat anti-rabbit IgG (HRP) antibody (Abcam, ab6721) secondary antibody to incubate at room temperature for 1 h, washing 4 times with TBST, placing the PVDF membrane on a clean imaging plate, then adding Immobilon Western HRP Substrate (MILLIPORE, WBKLS0100) for color development, photographing under a biomolecular imager, and performing quantitative analysis with Image J.

The results show that in the brain homogenate of Parkinson's disease model mice, the amount of Pro-BDNF in the plasminogen group is significantly lower than that in the vehicle control group, and the difference is significant (* represents P<0.05, *** represents P<0.001); the amount of BDNF in the plasminogen group is significantly higher than that in the vehicle control group, and the difference is extremely significant (Fig. 4). It is suggested that plasminogen can promote the cleavage of Pro-BDNF and the formation of mature BDNF in brain homogenate of Parkinson's disease model mice.

**Example 5: Plasminogen Promotes Cleavage of Pro-BDNF in Brain Homogenate of Alzheimer's Disease Model Mice**

Four B6SJLTg (APPSwFILon, PSEN1*M146L*L286V) 6799Vas/Mmjax (FAD) (Stock Number: 034840) (abbreviated as FAD) mice of 11 week-old were selected, and after sacrifice the whole brain tissue was taken out to weigh and place in Eppendorf ( EP) tubes, 1$\times$PBS (Thermo Fisher, pH 7.4; 10010-031) was respectively added at 150mg tissue/mL PBS to homogenize at 4°C (1min/time, 3-4 times), after homogenization, centrifuging at 4°C (12000rpm, 15min) to take the supernatant brain homogenate to place it into a new EP tube.

Eppendorf (EP) tubes were taken to set them as: ①blank control group, ②vehicle control group, and③plasminogen group, with 5 parallel sets in each group. Particularly, 21.5 $\mu$L of normal saline, 4.6 $\mu$L of plasminogen solution (2mg/mL), and 23.9 $\mu$L of mouse brain homogenate were added in the blank control group; 21.5 $\mu$L of A$\beta$40 (Shanghai QyaoBio, 04010011521, 1.0mg/mL), 4.6$\mu$L of vehicle solution (citric acid-sodium citrate solution), 23.9$\mu$L of mouse brain homogenate were added in the vehicle control group; 21.$\mu$L of A$\beta$40 (1.0mg/mL), 4.6$\mu$L of plasminogen solution (2mg/mL),

23.9μL of mouse brain homogenate were added in the plasminogen group. After the samples were added in each group, incubating at 37°C for 6 h, then 100 μL of 0.1% trifluoroacetic acid solution was added to terminate the reaction.

A 12% gel was prepared according to the SDS-PAGE gel formulation instructions. Samples in each group was respectively mixed well with 4× loading buffer (TaKaRa, e2139) at a volume ratio of 3:1, then heating at 100°C for 5 min, cooling to centrifuge for 2 min, and then 20 μL was taken for loading. The electrophoresis condition is 30V to run for 45min, then 100V electrophoresis to the bottom of the gel. After electrophoresis, the gel was stripped and stained with 1‰ Coomassie Brilliant Blue staining solution (1g Coomassie Brilliant Blue R250 dissolved in 1000ml mixture of ethanol: glacial acetic acid: purified water at a volume ratio of 5:2:13) for 30min, then decolorizing until clean by decolorization solution (a mixture of purified water: glacial acetic acid: absolute ethanol = a volume ratio of 17:2:1). The gel was photographed under a biomolecular imager, and quantitatively scanned for analysis.

The results show that in the brain homogenate of Alzheimer's disease model mice, the amount of Pro-BDNF in the plasminogen group is significantly lower than that in the vehicle control group, and the difference is extremely significant (*** represents P<0.001) (Fig. 5). It is suggested that plasminogen can promote cleavage of Pro-BDNF in brain homogenate of Alzheimer's disease model mice.

## Example 6: Plasminogen Promotes Cleavage of Pro-BDNF to Form Mature BDNF in the Brain Homogenate of Alzheimer's Disease Model Mice

Four B6SJLTg (APPSwFlLon, PSEN1*M146L*L286V) 6799Vas/Mmjax (FAD) (Stock Number: 034840) (abbreviated as FAD) mice of 11 week-old were selected, and after sacrifice the whole brain tissue was taken out to weigh and place in Eppendorf ( EP) tubes, 1×PBS (Thermo Fisher, pH 7.4; 10010-031) was respectively added at 150mg tissue/mL PBS to homogenize at 4°C (1min/time, 3-4 times), after homogenization, centrifuging at 4°C (12000rpm, 15min) to take the supernatant brain homogenate to place it into a new EP tube.

Eppendorf (EP) tubes were taken to set them as: ①blank control group, ②vehicle control group, and ③plasminogen group, with 5 parallel sets in each group. Particularly, 21.5 μL of normal saline, 4.6 μL of plasminogen solution (2mg/mL), and 23.9 μL of mouse brain homogenate were added in the blank control group; 21.5 μL of Aβ40 (Shanghai QyaoBio, 04010011521, 1.0mg/mL), 4.6μL of vehicle solution (citric acid-sodium citrate solution), 23.9μL of mouse brain homogenate were added in the vehicle control group; 21.μL of Aβ40 (1.0mg/mL), 4.6μL of plasminogen solution (2mg/mL), 23.9μL of mouse brain homogenate were added in the plasminogen group. After the samples were added in each group, incubating at 37°C for 6 h, then 100 μL of 0.1% trifluoroacetic acid solution was added to terminate the reaction.

A 12% gel was prepared according to the SDS-PAGE gel formulation instructions. Samples in each group was respectively mixed well with 4× loading buffer (TaKaRa, e2139) at a volume ratio of 3:1, then heating at 100°C for 5 min, cooling to centrifuge for 2 min, and then 20 μL was taken for loading. The electrophoresis condition is 30V to run for 45min, then 100V electrophoresis to the bottom of the gel. After the electrophoresis, the gel was stripped and transferred to a PVDF membrane (GE, A29433753), and the electrophoresis condition was 15V for 2.5h. The transferred PVDF membrane was soaked in blocking solution (5% skim emulsion) to block overnight in a refrigerator at 4°C, washing 4 times with TBST (0.01M Tris-NaCl, pH7.6 buffer), and then adding rabbit anti-human BDNF antibody (Boster Biological Technology, PB9075) to incubated at room temperature for 3 h, washing 4 times with TBST, adding goat anti-rabbit IgG (HRP) antibody (Abcam, ab6721) secondary antibody to incubate at room temperature for 1 h, washing 4 times with TBST, and placing the PVDF membrane on a clean imaging plate, then adding Immobilon Western HRP Substrate (MILLIPORE, WBKLS0100) for color development, photographing under a biomolecular imager, and performing quantitative analysis on the optical density of the bands with Image J.

The results show that in the brain homogenate of Alzheimer's disease model mice, the amount of Pro-BDNF in the plasminogen group is significantly lower than that in the vehicle control group, and the difference is extremely significant (** represents P<0.01, *** represents P <0.001); the amount of BDNF in the plasminogen group is significantly higher than that in the vehicle control group, and the difference is extremely significant (Fig. 6). It is suggested that plasminogen can promote cleavage of Pro-BDNF and the formation of mature BDNF in brain homogenate of Alzheimer's disease model mice.

## Example 7: Administration of Plasminogen Promotes Increase of Plasminogen Level in the Brain Tissue of SMA Mice

Eight FVB.Cg-Grm7Tg(SMN2)89Ahmb Smn1tm1MsdTg(SMN2*delta7)4299Ahmb/J gene mutant mice (referred to as SMNΔ7SMA mice) of 3- or 7-day-old (purchased from Jackson Laboratory, pedigree number: 005025) and four wild-type mice of the same age were taken, particularly, SMNΔ7 SMA mice were randomly divided into vehicle group and plasminogen group with 4 mice in each group, and 4 wild-type mice were used as blank control group. The mice in the plasminogen group were intraperitoneally injected with plasminogen at 60 mg/kg/day at a concentration of 10 mg/ml, and the mice in the vehicle group and the blank control group were injected with vehicle at 6 ml/kg/day, all mice were administrated until day 11 after birth. All mice were sacrificed and dissected 2 hours after administration on day 11 after birth, and part of the brain tissue was taken to homogenize for detection according to the instructions of the Human Plasminogen ELISA Kit (manufacturer: AssayMax, catalog number: EP1200-1). The human plasminogen working standard was used as the internal standard, the concentration of each sample was calibrated, and the calibrated concentration was divided by the total protein concentration to calculate the amount of plasminogen in the total protein amount of each sample unit, and carry out statistical analysis.

SMNΔ7 SMA mice have homozygous mutations in the SMN1 gene, and express the human SMN2 gene. The clinical and pathological manifestations of these mice are similar to those of human spinal muscular atrophy (SMA).

The results show that the plasminogen level in the brain tissue of wild-type mice in the blank control group is 1.18±1.54ng/mg; the plasminogen level in the brain tissue of mice in the vehicle group is 1.49±1.59ng/mg, the plasminogen level in the brain tissue of the mice in the vehicle group does not change significantly as compared with the blank control group; the plasminogen level in the SMA transgenic mice in the plasminogen group is 12.09±5.32ng/mg, about 8 times that of mice in the vehicle group (Fig. 7). It is suggested that supplementing plasminogen can promote the permeability of blood-brain barrier to plasminogen under SMA disease conditions, and plasminogen can pass through the blood-brain barrier to reach the brain.

**Example 8: Administration of Plasminogen Promotes Increase of Plasminogen Level in the Spinal Cord Tissue of SMA Mice**

Eight SMNΔ7 SMA mice of 3- or 7-day-old, four wild-type mice of the same age, and three SMA heterozygous mice of the same age were taken. The SMNΔ7 SMA mice were randomly divided into vehicle group and plasminogen group with 4 mice in each group; 4 wild-type mice were used as the blank control group, and 3 SMA heterozygous mice were used as the normal plasminogen administration control group. The mice in the plasminogen group and the normal plasminogen administration control group were intraperitoneally injected with plasminogen at 60 mg/kg/day at a concentration of 10 mg/ml, and the mice in the vehicle group and the blank control group were intraperitoneally injected with vehicle at 6 ml/kg/day, all the mice were administrated until day 11 after birth. All the mice were sacrificed and dissected 2 hours after the administration on day 11 after birth, and part of the spinal cord tissue was collected to homogenize for plasminogen ELISA detection.

The results show that the plasminogen level of spinal cord of wild-type mice in the blank control group is 8.51±9.51ng/mg. The plasminogen level of spinal cord of SMA transgenic mice in the vehicle group is 19.95±4.06ng/mg, which was slightly higher than that of mice in the blank control group. The plasminogen level in the normal plasminogen administration control group is 13.03±7.51ng/mg. The plasminogen level of spinal cord of the SMA transgenic mice in the plasminogen group is 62.33±17.37ng/mg, which is about three times that of mice in the vehicle group, and the statistical analysis P value as compared between the plasminogen group and the vehicle group is 0.029, which is 4.8 times that of the normal plasminogen administration control group, and the statistical analysis P value as compared between the plasminogen group and the normal plasminogen administration control group is 0.026 (Fig. 8). It is suggested that the administration of plasminogen can promote increase of the permeability of the blood-spinal cord barrier to plasminogen under the condition of SMA, and the plasminogen can pass through the blood-spinal cord barrier to reach the spinal cord.

**Example 9: Administration of Plasminogen Promotes Increase of the Plasminogen Level in Spinal Cord Tissue of LPS-induced Pneumonia SMA Heterozygous Mice**

Nine SMA heterozygous mice (purchased from Jackson Laboratory, pedigree number: 005025) were randomly divided into 2 groups according to body weight, particularly 3 mice in the blank control group and 6 mice in the model group. After all the mice were anesthetized with Zoletil 50, the mice in the model group were tracheal instilled with 2.5 mg/ml bacterial lipopolysaccharide (LPS) solution for modeling, and the dose for modeling was 5 mg/kg, the mice in the blank control group were tracheal instilled with 2ml/kg normal saline. After 2 hours of modeling, all mice in the model group were randomly divided into two groups according to body weight, particularly 3 mice in the vehicle group and 3 mice in the plasminogen group. After the grouping was completed, the mice were administered, and the mice in the plasminogen group were given plasminogen by tail vein injection at 50 mg/kg/mouse, and mice in vehicle group and blank control group were given vehicle by tail vein injection at 5ml/kg/mouse. All mice were sacrificed 2 hours after administration, and spinal cord tissues were dissected to collect and homogenate for detection according to the instructions of Human Plasminogen ELISA Kit (manufacturer: AssayMax, catalog number: EP1200-1). The human plasminogen working standard was used as the internal standard, the concentration of each sample was calibrated, and the calibrated concentration was divided by the total protein concentration to calculate the amount of plasminogen in the total protein amount of each sample unit, and carry out statistical analysis.

The results show that the plasminogen level in spinal cord tissue homogenate of mice in the blank control group is 2.70±0.74ng/mg; after tracheal instillation of LPS, the plasminogen level in spinal cord tissue homogenate of mice in vehicle group is 3.17±1.51ng/mg, there was no significant change as compared with mice in the blank control group; after the mice in the plasminogen group were supplemented with 2.5 times the physiological dose of plasminogen, the plasminogen level is 121. 16±44.68ng/mg, which is about 38.2 times that of the mice in the vehicle group (Fig. 9). It is suggested that supplementing plasminogen can promote the permeability of blood-spinal cord barrier to plasminogen in LPS-induced pneumonia SMA heterozygous mice. Under this condition, plasminogen can pass through the blood-spinal cord barrier to enter the central nervous system.

**Example 10: Administration of Plasminogen Promotes Increase of the Plasminogen Level in Spinal Cord Tissue of LPS-induced Pneumonia SMA Heterozygous Mice**

Nine SMA heterozygous mice (purchased from Jackson Laboratory, pedigree number: 005025) were randomly divided into 2 groups according to body weight, particularly 3 mice in the blank control group and 6 mice in the model group. After all the mice were anesthetized with Zoletil 50, the mice in the model group were tracheal instilled with 2.5 mg/ml bacterial lipopolysaccharide (LPS) solution for modeling, and the dose for modeling was 5 mg/kg, the mice in the blank control group were tracheal instilled with 2ml/kg normal saline. After 2 hours of modeling, all mice in the model group were randomly divided into two groups according to body weight, particularly 3 mice in the vehicle group and 3 mice in the plasminogen group. After the grouping was completed, the mice were administered, and the mice in the plasminogen group were given plasminogen by tail vein injection at 50 mg/kg/mouse, and mice in vehicle group and blank control group were given vehicle by tail vein injection at 5ml/kg/mouse. All the mice were sacrificed 2 hours after the administration, and the spinal cord tissues were dissected to collect and homogenate for detection of plasminogen by enzyme-substrate kinetics method. 85 μL/well of standard solution, blank, and sample were added at seven different concentration points to the microplate (manufacturer: NUNC, catalog number: 446469) successively, and then adding a mixture of 15 μL of 20mM S-2251 solution (manufacturer: Chromogenix, catalog number: 82033239) and 100ng/μL uPA solution (mixed at a volume ratio of 2:1 just before use) to incubate at 37°C. From 0 min of reaction, the A405 absorbance value was read in a multi-functional microplate reader every 5 min, until the reaction was performed for 90 min. All the reactions were fitted with a straight line with time and absorbance value, and the slope of the straight line was the reaction rate (ΔA405/min) of the standard substance/sample. Finally, the potency value of the standard substance and ΔA405/min were used as the standard curve to calculate the potency of the tested samples. The plasminogen activity in the total protein amount of each sample unit was calculated.

The results show that the plasminogen level in the spinal cord of mice in the blank control group is 0.00011±4.51×10⁻⁵ U/mg; after tracheal instillation of LPS, the plasminogen level in the spinal cord of mice in the vehicle group was 0.00010±9.72×10⁻⁶ U/mg, and there was no significant change as compared with mice in the blank control group; after the mice in the plasminogen group were supplemented with 2.5 times the physiological dose of plasminogen, the plasminogen level increased to 0.00034±1.04×10⁻⁴ U/mg, the statistical analysis P value is 0.058 (Fig. 10) as compared with that of mice in the vehicle group. It is suggested that supplementing plasminogen can promote the permeability of blood-spinal cord barrier to plasminogen in LPS-induced pneumonia SMA heterozygous mice, and plasminogen can pass through the blood-spinal cord barrier to accumulate in the spinal cord.

**Example 11: Administration of Plasminogen Promotes Increase of the Plasminogen Level in Spinal Cord Tissue of LPS-induced Pneumonia Mice**

Fifteen C57 male mice were weighed and randomly divided into two groups, particularly 3 mice in the blank control group, and 12 mice in the model group. All mice in the model group were anesthetized with Zoletil 50, and then 2.5 mg/ml LPS solution was tracheal instilled for modeling, and the dose for modeling was 5 mg/kg. After 2 hours of modeling, all mice in the model group were divided into four groups according to body weight, particularly 3 mice in the vehicle group, 3 mice in the plasminogen group A, 3 mice in the plasminogen group B, and 3 mice in the plasminogen group C. After the grouping was completed, the administration began. The mice in the plasminogen group A were injected with plasminogen by tail vein at 50 mg/kg/mouse, the mice in the plasminogen group B were injected with plasminogen by tail vein at 17 mg/kg/mouse, and the mice in the plasminogen group C were injected with plasminogen by tail vein at 6 mg/kg/mouse. Mice in the vehicle group and mice in the blank control group were injected with the vehicle by tail vein at 5ml/kg/mouse. 2 hours after administration, spinal cord tissue was dissected to collect for specific human plasminogen detection by ELISA method.

The results of ELISA detection of plasminogen level in the spinal cord tissue homogenate show that, the Plg content of the spinal cord tissue of mice in the blank control group is 7.71±0.51ng/mg, and the Plg content of the spinal cord tissue of the mice in the vehicle group is 14.04±3.25ng/mg. After tracheal instillation of LPS, the plasminogen level in the spinal cord of the mice is increased; after supplementation of 50 mg/kg (about 1 mg per mouse) of plasminogen to the mice in the plasminogen group A, the plasminogen level in the spinal cord tissue is 85.10±11.59 ng/mg, which is about 6.1 times that of the mice in the vehicle group; after supplementation of 17mg/kg (about 0.34mg per mouse) of plasminogen to the mice in the plasminogen group B, the plasminogen level in spinal cord tissue is 77.90± 21.39ng/mg, which is about 5.5 times that of the mice in the vehicle group; after supplementation of 6mg/kg (about 0.1mg per mouse) of plasminogen to the mice in the plasminogen group C, the plasminogen level in spinal cord tissue is 64.00±19.63ng/mg, which is about 4.6 times that of the mice in the vehicle group (Fig. 11). It is suggested that: 1) supplementing plasminogen can promote the permeability of blood-brain barrier to plasminogen in LPS-induced pneumonia mice, and plasminogen can pass through the blood-brain barrier to enrich in spinal cord tissue; 2) the enrichment of plasminogen in the spinal cord has a dose-dependent effect, within the dose range of 6-50mg/kg, the enrichment plasminogen level in the spinal cord increases with the increase of the administration dose of plasminogen.

**Example 12: Administration of Plasminogen Promotes Increase of the Plasminogen Level in Spinal Cord Tissue of LPS- Induced Pneumonia Mice**

Fifty-four C57 male mice of 6-9 week-old were weighed and randomly divided into two groups, particularly 15 mice in the blank control group, and 39 mice in the model group. After all the mice were anesthetized with Zoletil 50, the mice in the model group were tracheal instilled with 2.5 mg/ml LPS solution for modeling, and the dose for modeling was 5 mg/kg, and the mice in the blank group were tracheal instilled with 2 ml/kg of normal saline. After 2 hours of modeling with LPS, all the mice in the model group were divided into three groups according to body weight, particularly 15 mice in the LPS+vehicle group, 12 mice in the LPS+50mg/kg plasminogen group, and 12 mice in the LPS+6mg/kg plasminogen group. The mice in the blank group were randomly divided into two groups according to body weight, particularly 3 mice in the blank+vehicle group, and 12 mice in the blank+50mg/kg plasminogen group, and the administration was started. LPS+50mg/kg plasminogen group and blank+50mg/kg plasminogen group were given plasminogen by tail vein injection at 50mg/kg body weight, LPS+vehicle group and blank+vehicle group were given vehicle by tail vein injection at 5ml/kg/mouse, and the LPS+6mg/kg plasminogen group was given plasminogen by tail vein injection at 6mg/kg body weight. Three mice in blank+vehicle group and three mice in LPS+vehicle group were sacrificed at 0 hour after administration, and three mice in each group were randomly sacrificed at each time point of 2, 6, 12 and 24 hours. After sacrifice the spinal cord was collected to homogenize for specific human plasminogen detection by ELISA method.

The results show that mice in the sham operation+vehicle group and LPS+vehicle group do not receive plasminogen injection, and no human plasminogen is detected in the spinal cord tissue homogenate at 0, 2, 6, 12 and 24 hours. Mice in the sham operation+50mg/kg plasminogen group and LPS+50mg/kg plasminogen group are injected with 50mg/kg body weight of plasminogen, 2 hours after injection human plasminogen in spinal cord tissue homogenate is increased significantly, and 6-12 hours after injection the plasminogen level is decreased significantly, and human plasminogen is basically undetectable at 24 hours. Mice in the LPS+6mg/kg plasminogen group are injected with plasminogen at 6mg/kg body weight, and 2 hours after injection the plasminogen level detected in spinal cord tissue homogenate is significantly higher than that of mice in the LPS+vehicle group, and is significantly lower than that of mice in the LPS+50mg/kg plasminogen group (Fig. 12).

The results indicate that: 1) after administration of plasminogen under physiological and pathological conditions, it can promote plasminogen to pass through the blood-brain barrier to enrich in spinal cord tissue; 2) intravenous injection of plasminogen in normal mice *in vivo,* the plasminogen level in spinal cord tissue is increased significantly; 3) the enrichment of plasminogen in the spinal cord has a time-dependent effect, which is increased firstly, then gradually decreased in 2-12 hours, and the metabolism is almost completely finished in 12-24 hours; 4) the enrichment of plasminogen in the spinal cord has a dose-dependent effect, the higher the administration dose, the higher the plasminogen level in the spinal cord tissue.

**Example 13: Administration of Plasminogen Promotes Increase of the Plasminogen Level in Brain Tissue of LPS-Induced Pneumonia Mice**

Fifty-four C57 male mice of 6-9 week-old were weighed and randomly divided into two groups, particularly 15 mice in the blank control group, and 39 mice in the model group. After all the mice were anesthetized with Zoletil 50, the mice in the model group were tracheal instilled with 2.5 mg/ml LPS solution for modeling, and the dose for modeling was 5 mg/kg, and the mice in the blank group were tracheal instilled with 2 ml/kg of normal saline. After 2 hours of modeling with LPS, all the mice in the model group were divided into three groups according to body weight, particularly 15 mice in the LPS+vehicle group, 12 mice in the LPS+50mg/kg plasminogen group, and 12 mice in the LPS+6mg/kg plasminogen group. The mice in the blank group were randomly divided into two groups according to body weight, particularly 3 mice in the blank+vehicle group, and 12 mice in the blank+50mg/kg plasminogen group, and the administration was started. LPS+50mg/kg plasminogen group and blank+50mg/kg plasminogen group were given plasminogen by tail vein injection at 50mg/kg body weight, LPS+vehicle group and blank+vehicle group were given vehicle by tail vein injection at 5ml/kg/mouse, and the LPS+6mg/kg plasminogen group was given plasminogen by tail vein injection at 6mg/kg body weight. Three mice in blank+vehicle group and three mice in LPS+vehicle group were sacrificed at 0 hour after administration, and three mice in each group were randomly sacrificed at each time point of 2, 6, 12 and 24 hours. After sacrifice the brain was collected to homogenize for specific human plasminogen detection by ELISA method.

The results show that mice in the blank+vehicle group and LPS+vehicle group do not receive plasminogen injection, and no human plasminogen is detected in the brain tissue homogenate at 0, 2, 6, 12 and 24 hours. Mice in blank+50mg/kg plasminogen group and LPS+50mg/kg plasminogen group are injected with 50mg/kg body weight of plasminogen, 2 hours after injection human plasminogen in brain tissue homogenate is detected to increase significantly, and 6-12 hours after injection the plasminogen level is decreased significantly, and human plasminogen is basically undetectable at 24 hours. Mice in the LPS+6mg/kg plasminogen group are injected with plasminogen at 6mg/kg body weight, and 2 hours after injection the plasminogen level detected in brain tissue homogenate is significantly higher than that of mice in the LPS+vehicle group, and is significantly lower than that of mice in the LPS+50mg/kg plasminogen group (Fig. 13).

The results indicate that: 1) after administration of plasminogen under physiological and pathological conditions, it can promote plasminogen to pass through the blood-brain barrier to enrich in brain tissue; 2) intravenous injection of plasminogen in normal mice *in vivo,* the plasminogen level in brain tissue is increased significantly; 3) the enrichment of plasminogen in brain tissue has a time-dependent effect, which is increased firstly, then gradually decreased in 2-12 hours, and the metabolism is almost completely finished in 12-24 hours; 4) the enrichment of plasminogen in brain tissue has a dose-dependent effect, the higher the administration dose, the higher the plasminogen level in brain tissue.

**Example 14: Administration of Plasminogen Promotes Increase of the Plasminogen Level in Spinal Cord Tissue of SOD1-G93A Mice**

Twenty seven B6.Cg-Tg(SOD1-G93A)1Gur/J transgenic mice (hereinafter referred to as SOD1-G93A mice) of 15 week-old (purchased from Jackson Laboratory, pedigree number: 004435) were randomly divided into three groups, particularly 3 mice in the vehicle group, 12 mice in the 50mg/kg plasminogen group, and 12 mice in the 6mg/kg plasminogen group. The mice in the vehicle group were given with vehicle by tail vein injection, mice in the 50mg/kg plasminogen group was given plasminogen by tail vein injection at 50mg/kg body weight, and the 6mg/kg plasminogen group was given plasminogen by tail vein injection at 6mg/kg body weight. 2 hours after administration, the mice in the vehicle group were sacrificed, and 3 mice in each group of the remaining mice were sacrificed at 2, 6, 12, and 24 hours after administration. After sacrifice, the spinal cord was collected to homogenize for specific human plasminogen detection by ELISA method.

SOD1-G93A mice overexpress the human mutant SOD1 gene, and the clinical and pathological manifestations of the mice are similar to human amyotrophic lateral sclerosis.

The results of ELISA detection of the spinal cord of SOD1-G93A mice show that: the plasminogen level in the spinal cord of SOD1-G93A mice is significantly increased after tail vein injection of 50 mg/kg and 6 mg/kg plasminogen, and the plasminogen level of mice in the 50 mg/kg plasminogen group is significantly higher than that of mice in the 6mg/kg group. 2 hours after administration, the plasminogen level is gradually decreased, and its metabolism is basically completely finished in 12-24 hours (Fig. 14).

The results indicate that: 1) the plasminogen administered at a physiological dose level can pass through the blood-brain barrier of SOD1-G93A mice to enrich in the spinal cord tissue; 2) the enrichment of plasminogen in the spinal cord has a dose-dependent effect, and the higher the administration dose of plasminogen, the more enriched; 3) the enrichment of plasminogen in the spinal cord has a time-dependent effect, which is increased firstly, then gradually decreased in 2-12 hours, and its metabolism is basically completely finished in 12-24 hours.

**Example 15: Administration of Plasminogen Promotes Increase of the Plasminogen Level in Brain Tissue of SOD1-G93A Mice**

Twenty seven SOD1-G93A mice of 15 week-old were randomly divided into three groups, particularly 3 mice in the vehicle group, 12 mice in the 50mg/kg plasminogen group, and 12 mice in the 6mg/kg plasminogen group. The mice in the vehicle group were given with vehicle by tail vein injection, mice in the 50mg/kg plasminogen group was given plasminogen by tail vein injection at 50mg/kg body weight, and the 6mg/kg plasminogen group was given plasminogen by tail vein injection at 6mg/kg body weight. 2 hours after administration, the mice in the vehicle group were sacrificed, and 3 mice in each group of the remaining mice were sacrificed at 2, 6, 12, and 24 hours after administration. After sacrifice, the brain was collected to homogenize for specific human plasminogen detection by ELISA method.

The results of ELISA level detection of the brain of SOD1-G93A mice show that: the plasminogen level in the brain of SOD1-G93A mice is significantly increased after tail vein injection of 50 mg/kg and 6 mg/kg plasminogen, and the plasminogen level of mice in the 50 mg/kg plasminogen group is significantly higher than that of mice in the 6 mg/kg group. 2 hours after administration, the plasminogen level is gradually decreased, and its metabolism is basically completely finished in 12-24 hours (Fig. 15).

The results indicate that: 1) the plasminogen administered at a physiological dose level can pass through the blood-brain barrier of SOD1-G93A mice to enrich in the brain tissue; 2) the enrichment of plasminogen in the brain tissue has a dose-dependent effect, and the higher the administration dose of plasminogen, the more enriched; 3) the enrichment of plasminogen in the brain tissue has a time-dependent effect, which is increased firstly, then gradually decreased in 2-12 hours, and its metabolism is basically completely finished in 12-24 hours.

**Example 16: Administration of Plasminogen Promotes Increase of the Plasminogen Level in Spinal Cord Tissue of FAD Mice**

Twenty seven B6-Tg (APPSwFILon, PSEN1*M146L*L286V) 6799Vas/Mmjax (hereinafter referred to as FAD mice) (purchased from Jackson Laboratory, pedigree number: 034840) were randomly divided into three groups, particularly 3 mice in the vehicle group, 12 mice in the 50mg/kg plasminogen group, and 12 mice in the 6mg/kg plasminogen group. The mice in the vehicle group were given with vehicle by tail vein injection, mice in the 50mg/kg plasminogen group was given plasminogen by tail vein injection at 50mg/kg body weight, and the 6mg/kg plasminogen group was given plasminogen by tail vein injection at 6mg/kg body weight. 2 hours after administration, the mice in the vehicle group were sacrificed, and 3 mice in each group of the remaining mice were sacrificed at 2, 6, 12, and 24 hours after administration. After sacrifice, the spinal cord was collected to homogenize for specific human plasminogen detection by ELISA method.

FAD mice are commonly used animal models in Alzheimer's disease research.

The results of ELISA level detection of spinal cord tissue homogenate show that, the plasminogen level in the spinal cord tissue of FAD mice is significantly increased after tail vein injection of 50 mg/kg and 6 mg/kg plasminogen, and the plasminogen level in the 50 mg/kg plasminogen group is significantly higher than that of mice in the 6mg/kg plasminogen group. 2 hours after administration, the plasminogen level is gradually decreased, and its metabolism is basically completely finished in 12-24 hours (Fig. 16).

The results indicate that: 1) the plasminogen administered at a physiological dose level can pass through the blood-brain barrier of FAD mice to enrich in the spinal cord tissue; 2) the enrichment of plasminogen in the spinal cord tissue has a dose-dependent effect, and the higher the administration dose of plasminogen, the more enriched; 3) the enrichment of plasminogen in the spinal cord tissue has a time-dependent effect, which is increased firstly, then gradually decreased in 2-12 hours, and its metabolism is basically completely finished in 12-24 hours.

**Example 17: Administration of Plasminogen Promotes Increase of the Plasminogen Level in Brain Tissue of FAD Mice**

Twenty seven FAD mice of 15 week-old were randomly divided into three groups, particularly 3 mice in the vehicle group, 12 mice in the 50mg/kg plasminogen group, and 12 mice in the 6mg/kg plasminogen group. The mice in the vehicle group were given with vehicle by tail vein injection, mice in the 50mg/kg plasminogen group was given plasminogen by tail vein injection at 50mg/kg body weight, and the 6mg/kg plasminogen group was given plasminogen by tail vein injection at 6mg/kg body weight. 2 hours after administration, the mice in the vehicle group were sacrificed, and 3 mice in each group of the remaining mice were sacrificed at 2, 6, 12, and 24 hours after administration. After sacrifice, the brain was collected to homogenize for specific human plasminogen detection by ELISA method.

The results of ELISA level detection of brain tissue homogenate show that, the plasminogen level in the brain tissue of FAD mice is significantly increased after tail vein injection of 50 mg/kg and 6 mg/kg plasminogen, and the plasminogen level in the 50 mg/kg plasminogen group is significantly higher than that of mice in the 6mg/kg plasminogen group. 2 hours after administration, the plasminogen level is gradually decreased, and its metabolism is basically completely finished in 12-24 hours (Fig. 17).

The results indicate that: 1) the plasminogen administered at a physiological dose level can pass through the blood-brain barrier of FAD mice to enrich in the brain tissue; 2) the enrichment of plasminogen in the brain tissue has a dose-dependent effect, and the higher the administration dose of plasminogen, the more enriched; 3) the enrichment of plasminogen in the brain tissue has a time-dependent effect, which is increased firstly, then gradually decreased in 2-12 hours, and its metabolism is basically completely finished in 12-24 hours.

**Example 18: Administration of Plasminogen Promotes BDNF Gene Transcription in Spinal Cord Tissue of SOD1-G93A Mice**

Female SOD1-G93A mice of 12 week-old were taken to start to observe the onset of the disease in detail. After onset of the disease, observation was started to record the onset time of each mouse when the legs tremble. 14 days after the onset of disease, the SOD1-G93A mice were randomly divided into two groups according to weight and behavioral tests, particularly 9 mice in the plasminogen group, and the other 9 mice in the vehicle group. Five wild-type (C57) mice of the same age were taken as the normal control group. The mice in the plasminogen group were injected with plasminogen by tail vein at 50mg/kg/day, and the mice in the vehicle group and the normal control group were injected with vehicle by tail vein at 5mL/kg/day. After administration for 29 consecutive days, the mice was sacrificed and dissected to collect spinal cord tissue. All BDNF gene transcripts were detected by qPCR.

The results show that the transcription level of BDNF gene in the spinal cord tissue of mice in the plasminogen group is significantly higher than that in the vehicle group, and the statistical difference P value is 0.05 (Fig. 18). It is suggested that plasminogen can promote the transcription of BDNF gene in the spinal cord of SOD1-G93A mice.

REFERENCES

[1] Gray K, Ellis V . Activation of pro-BDNF by the pericellular serine protease plasmin [J]. Febs Letters, 2008, 582(6):907-910.

[2] Kowianski, Przemys?aw, Lietzau G , Czuba E , et al. BDNF: A Key Factor with Multipotent Impact on Brain Signaling and Synaptic Plasticity[J]. Cellular & Molecular Neurobiology, 2017

[3] Cuprizone Model as a Tool for Preclinical Studies of the Efficacy of Multiple Sclerosis Diagnosis and Therapy[J]. Bulletin of Experimental Biology and Medicine, 2015, 159(1):111-115.

[4] Komoly S . Experimental demyelination caused by primary oligodendrocyte dystrophy. Regional distribution of the lesions in the nervous system of mice [corrected].[J]. ideggyógyászati szemle, 2005.

**Claims**

1. A method for promoting the formation of mature BDNF and/or increasing BDNF level, comprising: administering to a subject a therapeutically effective amount of a plasminogen pathway activator.

2. The method according to claim 1, wherein the plasminogen pathway activator promotes cleavage of Pro-BDNF to form mature BDNF and BDNF gene transcription or expression in the nerve tissue of the subject.

3. The method according to claim 1 or 2, wherein the plasminogen pathway activator increases the gene transcription, protein expression and level of an additional neurotrophic factor in the nerve tissue of the subject.

4. The method according to claim 3, wherein the additional neurotrophic factor comprises nerve growth factor (NGF), neurotrophic factor 3 (NT-3), neurotrophic factor 4/5 (NT-4/5), ciliary neurotrophic factor (CNTF), glial cell line-derived neurotrophic factor (GDNF), leukemia inhibitory factor (LIF), insulin-like growth factor-1 (IGF-1), transforming growth factor (TGF), epidermal growth factor (EGF), fibroblast growth factor (FGF) or platelet-derived growth factor (PDGF).

5. The method according to any one of claims 1-4, wherein the plasminogen pathway activator has one or more uses or activities selected from the group consisting of: promoting the survival, differentiation, growth and development of neurons; preventing neurons from being injured and dying; promoting the regeneration and differentiation of injured neurons; and maintaining neuron survival and normal physiological function.

6. The method according to any one of claims 1-5, wherein the subject is a subject suffering from injury of nerve or brain tissue caused by one or more diseases or conditions selected from the group consisting of:

   1) infection, selected from one or more of the following: meningitis, encephalitis, poliomyelitis and epidural abscess;
   2) vascular diseases, selected from one or more of the following: stroke, transient ischemic attack (TIA), subarachnoid hemorrhage, subdural hemorrhage and hematoma, and epidural hemorrhage;
   3) nerve structural injury diseases, selected from one or more of the following: brain or spinal cord injury, Bell palsy, cervical spondylosis, carpal tunnel syndrome, brain or spinal cord tumor, peripheral neuropathy and Guillain-Barré syndrome;
   4) dysfunction, selected from one or more of the following: headache, epilepsy, insomnia, neuralgia, anxiety and depression;
   5) neurodegenerative diseases, selected from one or more of the following: Alzheimer's disease, Parkinson's disease, Huntington's disease (HD), amyotrophic lateral sclerosis (ALS), multiple sclerosis (MS), spinocerebellar ataxia, and Pick disease;
   6) motor neuron diseases, selected from one or more of the following: spinal muscular atrophy (SMA), progressive bulbar palsy, progressive muscle atrophy, and primary lateral sclerosis;
   7) tumors, selected from one or more of the following: brain tumor and brain cancer.

7. A method for preventing or treating a BDNF-associated disease or condition, comprising administering to a subject a therapeutically effective amount of a plasminogen pathway activator.

8. The method according to claim 7, wherein the BDNF-associated disease or condition comprises any one selected from the group consisting of:

1) infection, selected from any one of the following: meningitis, encephalitis, poliomyelitis and epidural abscess;

2) vascular diseases, selected from any one of the following: stroke, transient ischemic attack (TIA), subarachnoid hemorrhage, subdural hemorrhage and hematoma, and epidural hemorrhage;

3) nerve structural injury diseases, selected from any one of the following: brain or spinal cord injury, Bell palsy, cervical spondylosis, carpal tunnel syndrome, brain or spinal cord tumor, peripheral neuropathy and Guillain-Barré syndrome;

4) dysfunction, selected from any one of the following: headache, epilepsy, insomnia, neuralgia, anxiety and depression;

5) neurodegenerative diseases, selected from any of the following: Alzheimer's disease, Parkinson's disease, Huntington's disease (HD), amyotrophic lateral sclerosis (ALS), multiple sclerosis (MS), spinocerebellar ataxia and Pick disease;

6) motor neuron diseases, selected from any one of the following: spinal muscular atrophy (SMA), progressive bulbar palsy, progressive muscle atrophy, and primary lateral sclerosis;

7) tumors, selected from any one of the following: brain tumor and brain cancer;

8) peripheral neuropathy, nerve injury caused by trauma, optic neuropathy, polyneuritis, herpes zoster, facial paralysis, burn injury, bedsore, corneal ulcer, and side effects caused by radiotherapy or chemotherapy;

9) neuropsychiatric disorder, obsessive-compulsive disorder, post-traumatic stress disorder, anorexia nervosa, autoimmune diseases of the central nervous system, long-term or short-term memory disorder, children's learning disability, closed craniocerebral injury, attention deficit disorder, narcolepsy, sleep disorder, brain or nerve cell injury, and AIDS-related neurologic deficit, motor and convulsive disorder **characterized by** motor and/or vocal convulsion (for example, Tourette mental disorder, chronic motor or vocal convulsive disorder, short-term convulsive disorder and stereotypical movement disorder), substance abuse disorder (for example, substance dependence, substance abuse and sequelae of substance abuse/dependence, such as substance induced psychological disorder, substance withdrawal and substance induced dementia or amnesia), traumatic brain injury, tinnitus, ataxia, muscular rigidity (spasticity), neurotoxicity, mental retardation or cognitive impairment (e.g., non-syndromic X-linked mental retardation, fragile X syndrome, Down syndrome, autism) caused by alcohol or substance abuse (e.g., ecstasy, methamphetamine, etc.), aphasia, Bell palsy, Creutzfeldt-Jakob disease, encephalitis, age-related macular degeneration, ondine syndrome, WAGR syndrome, hearing loss, Reiter syndrome, optic nerve injury, diabetic neuropathy, complications of nerve transplantation, peripheral nerve injury, obesity, metabolic syndrome, asthma, atopic disease, allergic inflammation, eczema, neuroimmunologic disease or disorder, neuro otological disease or disorder, and aging related disease or disorder;

10) neuralgia, any one selected from the group consisting of: trigeminal neuralgia, chronic pain, chronic inflammatory pain, pain related to arthritis, fibromyalgia, pain related to cancer, pain related to digestive diseases, pain related to Crohn's disease, pain related to autoimmune disease, pain related to endocrine disease, pain related to diabetes neuropathy, phantom limb pain, spontaneous pain, chronic postoperative pain, chronic temporomandibular pain, burning pain, post-herpetic neuralgia, AIDS-related pain, type I and II complex regional pain syndrome, chronic back pain, pain related to spinal cord injury, pain related to drug intake and recurrent acute pain, neuropathic pain.

9. The method according to any one of claims 1-8, wherein the plasminogen pathway activator increases plasminogen level in nerve tissue of the subject.

10. The method according to any one of claims 1-9, wherein the plasminogen pathway activator is administered in combination with one or more other drugs or therapies.

11. The method according to any one of claims 1-10, wherein the plasminogen pathway activator is administered intravenously, intramuscularly, intrathecally, by nasal inhalation, aerosol inhalation, by nasal drop or eye drop.

12. The method according to any one of claims 1-11, wherein the plasminogen pathway activator is one or more selected from the group consisting of: a component of plasminogen activation pathway, a compound directly activating plasminogen or indirectly activating plasminogen by activating an upstream component of plasminogen activation pathway, a compound mimicking the activity of plasminogen or plasmin, a compound upregulating the expression of plasminogen or an activator of plasminogen, an analog of plasminogen, an analog of plasmin, an analog of tPA or uPA, and an antagonist of fibrinolysis inhibitor.

**13.** The method according to claim 12, wherein the component of plasminogen activation pathway is selected from the group consisting of: natural or recombinant plasminogen, human plasmin, Lys-plasminogen, Glu-plasminogen, plasmin, a variant of plasminogen and plasmin and the analog thereof comprising one or more kringle domains and/or protease domains of plasminogen and plasmin, mini-plasminogen, mini-plasmin, micro-plasminogen, micro-plasmin, delta-plasminogen, delta-plasmin, an activator of plasminogen, tPA and uPA.

**14.** The method according to claim 12, wherein the antagonist of fibrinolysis inhibitor is an antagonist of natural or recombinant PAI-1, complement C1 inhibitor, $\alpha$2 antiplasmin or $\alpha$2 macroglobulin, such as an antibody of PAI-1, complement C1 inhibitor, $\alpha$2 antiplasmin or $\alpha$2 macroglobulin.

**15.** The method according to any one of claims 1-13, wherein the plasminogen pathway activator is plasminogen.

**16.** The method according to claim 15, wherein the plasminogen comprises the amino acid sequence represented by SEQ ID NO: 2, 6, 8, 10 or 12; or comprises an amino acid sequence having at least 80%, 85%, 90%, 95%, 96%, 97%, 98% or 99% sequence identity with the amino acid sequence represented by SEQ ID NO: 2, 6, 8, 10 or 12, and has the proteolytic activity and/or lysine binding activity of plasminogen.

**17.** The method according to claim 15, wherein the plasminogen comprises one or more selected from the group consisting of:

1) a serine protease domain having the amino acid sequence represented by SEQ ID NO: 14;
2) a serine protease domain having at least 80%, 90%, 95%, 96%, 97%, 98%, 99% identity with SEQ ID NO: 14 and retaining proteolytic activity;
3) a Kringle domain selected from one or more of Kringle 1, Kringle 2, Kringle 3, Kringle 4 and Kringle 5; and
4) a Kringle domain having at least 80%, 90%, 95%, 96%, 97%, 98%, 99% identity with one or more selected from Kringle 1, Kringle 2, Kringle 3, Kringle 4 and Kringle 5, and retaining lysine-binding activity.

**18.** The method according to claim 15, wherein the plasminogen is selected from Glu-plasminogen, Lys-plasminogen, mini-plasminogen, micro-plasminogen, or delta-plasminogen, or a variant thereof retaining the proteolytic activity of plasminogen.

Fig. 1

Fig. 2

Fig. 3

Fig. 4

Fig. 5

Fig. 6

Fig. 7

Fig. 8

Fig. 9

Fig. 10

Fig. 11

Fig. 12

Fig. 13

Fig. 14

Fig. 15

Fig. 16

Fig. 17

Fig. 18

## INTERNATIONAL SEARCH REPORT

International application No.

**PCT/CN2021/131184**

| A. | CLASSIFICATION OF SUBJECT MATTER |
|---|---|

A61K 31/00(2006.01)i; A61P 25/00(2006.01)i; C12N 15/58(2006.01)i

According to International Patent Classification (IPC) or to both national classification and IPC

| B. | FIELDS SEARCHED |
|---|---|

Minimum documentation searched (classification system followed by classification symbols)

A61K; A61P; C12N

Documentation searched other than minimum documentation to the extent that such documents are included in the fields searched

Electronic data base consulted during the international search (name of data base and, where practicable, search terms used)

TWMED; TWTXT; USTXT; DWPI; ENTXTC; KRTXT; CNTXT; WPABSC; JPTXT; CNABS; EPTXT; CNMED; WPABS; ENTXT; WOTXT; CJFD; CATXT; VEN; genbank; 中国专利生物序列检索系统, Chinese Patent Biological Sequence Retrieval System: 纤溶酶原, plasmin , plasminogen, 神经因子, brain-derived neurotrophic factor, BDNF, 纤溶酶原, tissue plasminogen activator, tPA, 活化, activate, 激活, 裂解, 水解, cleavage, 催化, caly+, SEQ ID NO: 2, 6, 8, 10的序列检索, sequence search of SEQ ID NO: 2, 6, 8, 10.

| C. | DOCUMENTS CONSIDERED TO BE RELEVANT |
|---|---|

| Category* | Citation of document, with indication, where appropriate, of the relevant passages | Relevant to claim No. |
|---|---|---|
| X | 林志雄 等 (LIN, Zhixiong et al.). "血浆纤溶酶原与BDNF在抑郁模型大鼠海马区的表达及相关性研究 (Study on Plasminogen and Bdnf Expression in the Hippocampus of Depression Rat Mode and Its Relationship)" 重庆医学 (Chongqing Medical Journal), Vol. 45, No. 9, 13 May 2016 (2016-05-13), pp. 1170-1172, and see p. 1172, left column, paragraphs 4 and 5 | 1, 2, 5-9, 11-18 |
| Y | 林志雄 等 (LIN, Zhixiong et al.). "血浆纤溶酶原与BDNF在抑郁模型大鼠海马区的表达及相关性研究 (Study on Plasminogen and Bdnf Expression in the Hippocampus of Depression Rat Mode and Its Relationship)" 重庆医学 (Chongqing Medical Journal), Vol. 45, No. 9, 13 May 2016 (2016-05-13), pp. 1170-1172, and see p. 1172, left column, paragraphs 4 and 5 | 3, 4, 10 |
| X | PANG, P. T. et al. "Cleavage of proBDNF by tPA/Plasmin Is Essential for Long-Term Hippocampal Plasticity" Science, Vol. 306, No. 5695, 15 October 2004 (2004-10-15), pp. 487-491, and see abstract, p. 488, first column, last paragraph to second column, first paragraph, p. 489, last paragraph to p. 490, first paragraph, and p. 491, first paragraph | 1, 2, 5-9, 11-18 |

☑ Further documents are listed in the continuation of Box C.  ☑ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **28 January 2022** | **18 February 2022** |

| Name and mailing address of the ISA/CN | Authorized officer |
|---|---|
| **China National Intellectual Property Administration (ISA/CN)** **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**EP 4 248 954 A1**

**INTERNATIONAL SEARCH REPORT**

International application No.

**PCT/CN2021/131184**

**C.    DOCUMENTS CONSIDERED TO BE RELEVANT**

| Category* | Citation of document, with indication, where appropriate, of the relevant passages | Relevant to claim No. |
|---|---|---|
| Y | PANG, P. T. et al. "Cleavage of proBDNF by tPA/Plasmin Is Essential for Long-Term Hippocampal Plasticity" *Science,* Vol. 306, No. 5695, 15 October 2004 (2004-10-15), pp. 487-491, and see abstract, p. 488, first column, last paragraph to second column, first paragraph, p. 489, last paragraph to p. 490, first paragraph, and p. 491, first paragraph | 3, 4, 10 |
| Y | WO 9419461 A1 (CEPHALON INC.) 01 September 1994 (1994-09-01) description, p. 1, line 23 to p. 2, line 18, and embodiment 6 | 3, 4 |
| Y | 龙永春 等 (LONG, Yongchun et al.). "注射用鼠神经生长因子联合重组组织型纤溶酶原激活物静脉溶栓对急性脑梗死患者神经功能的影响 (Influence of Intravenous Thrombolysis with Mouse Nerve Growth Factor for Injection and Recombinant Tissue-Type Plasminogen Activator on Neurological Function of Patients with Acute Cerebral Infarction)" 实用临床医药杂志 *(Journal of Clinical Medicine in Practice),* Vol. 23, No. 11, 30 June 2019 (2019-06-30), pp. 7-10, and see abstract, and p. 10, left column, paragraph 2 | 10 |
| A | WO 2017028782 A1 (FUJIAN TIANTAI MEDICAL TECH CO LTD) 23 February 2017 (2017-02-23) see entire document | 1-18 |

Form PCT/ISA/210 (second sheet) (January 2015)

41

**INTERNATIONAL SEARCH REPORT**

| International application No. |
|---|
| **PCT/CN2021/131184** |

**Box No. I        Nucleotide and/or amino acid sequence(s) (Continuation of item 1.c of the first sheet)**

1.  With regard to any nucleotide and/or amino acid sequence disclosed in the international application, the international search was carried out on the basis of a sequence listing:

   a.  ☑ forming part of the international application as filed:

   ☑ in the form of an Annex C/ST.25 text file.

   ☐ on paper or in the form of an image file.

   b.  ☐ furnished together with the international application under PCT Rule 13*ter*.1(a) for the purposes of international search only in the form of an Annex C/ST.25 text file.

   c.  ☐ furnished subsequent to the international filing date for the purposes of international search only:

   ☐ in the form of an Annex C/ST.25 text file (Rule 13*ter*.1(a)).

   ☐ on paper or in the form of an image file (Rule 13*ter*.1(b) and Administrative Instructions, Section 713).

2.  ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3.  Additional comments:

Form PCT/ISA/210 (continuation of first sheet) (January 2015)

**INTERNATIONAL SEARCH REPORT**

| International application No. |
|---|
| **PCT/CN2021/131184** |

| Box No. II | Observations where certain claims were found unsearchable (Continuation of item 2 of first sheet) |
|---|---|

This international search report has not been established in respect of certain claims under Article 17(2)(a) for the following reasons:

1. ☑ Claims Nos.: **1-18**
   because they relate to subject matter not required to be searched by this Authority, namely:

   [1]  Claims 1-6 and 9-18 relate to a method for prompting mature BDNF formation and/or increasing a BDNF level, comprising administering a subject with a therapeutically effective dose of a plasminogen pathway activator; claims 7-18 relate to a method for preventing or treating BDNF-related diseases or symptoms, comprising administering a subject with a therapeutically effective dose of a plasminogen pathway activator, and therefore, the subject matter of claims 1-18 belongs to the subject matter defined in PCT Rule 39.1(iv) that does not warrant a search conducted by the international searching authority. The subject matter of claims 1-6 and 9-18 can be reasonably expected to be an "application of a plasminogen pathway activator in the preparation of drugs for prompting mature BDNF formation and/or increasing a BDNF level", and the subject matter of claims 7-18 can be reasonably expected to be an "application of a plasminogen pathway activator in the preparation of drugs for preventing or treating BDNF-related diseases or symptoms". The international search is provided on the basis of the reasonable expectations.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (January 2015)

INTERNATIONAL SEARCH REPORT
Information on patent family members

International application No.

**PCT/CN2021/131184**

| Patent document cited in search report | | | Publication date (day/month/year) | Patent family member(s) | | | Publication date (day/month/year) |
|---|---|---|---|---|---|---|---|
| WO | 9419461 | A1 | 01 September 1994 | None | | | |
| WO | 2017028782 | A1 | 23 February 2017 | CN | 106466479 | A | 01 March 2017 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 102154253 A
- US 3773919 A
- EP 58481 A

### Non-patent literature cited in the description

- **NY, A. ; LEONARDSSON, G. ; HAGGLUND, A.C ; HAGGLOF, P. ; PLOPLIS, V.A. ; CARMELIET, P. ; NY, T.** Ovulation inplasminogen-deficient mice. *Endocrinology,* 1999, vol. 140, 5030-5035
- **SILVERSTEIN RL ; LEUNG LL ; HARPEL PC ; NACHMAN RL.** Complex formation of platelet thrombospondin with plasminogen. Modulation of activation by tissue activator. *J. Clin. Invest.,* November 1984, vol. 74 (5), 1625-33
- **GRAVANIS I ; TSIRKA SE.** Tissue-type plasminogen activator as a therapeutic target in stroke. *Expert Opinion on Therapeutic Targets,* February 2008, vol. 12 (2), 159-70
- **GEIGER M ; HUBER K ; WOJTA J ; STINGL L ; ESPANA F ; GRIFFIN JH ; BINDER BR.** Complex formation between urokinase and plasma protein C inhibitor in vitro and in vivo. *Blood,* August 1989, vol. 74 (2), 722-8
- **AISINA RB ; MUKHAMETOVA L I.** Structure and function of plasminogen/plasmin system [J. *Russian Journal of Bioorganic Chemistry,* 2014, vol. 40 (6), 590-605
- *Barany and Solid-Phase Peptide Synthesis,* 3-284
- Special Methods in Peptide Synthesis, Part A. *The Peptides: Analysis, Synthesis, Biology,* vol. 2
- **MERRIFIELD et al.** *J. Am. Chem. Soc.,* 1963, vol. 85, 2149-2156
- **STEWART et al.** Solid Phase Peptide Synthesis. Pierce Chem. Co, 1984
- **GANESAN A.** *Mini Rev. Med Chem.,* 2006, vol. 6, 3-10
- **CAMARERO JA et al.** *Protein Pept Lett.,* 2005, vol. 12, 723-8
- **WINNACKER.** From Genes to Clones. VCH Publishers, 1987
- **QUEEN et al.** *Immunol. Rev.,* 1986, vol. 89, 49
- **CO et al.** *J. Immunol.,* 1992, vol. 148, 1149
- Remington's Pharmaceutical Sciences. 1980
- **LANGER et al.** *J. Biomed. Mater. Res.,* 1981, vol. 15, 167-277
- **LANGER.** *Chem. Tech.,* 1982, vol. 12, 98-105
- **SIDMAN et al.** *Biopolymers,* 1983, vol. 22, 547
- **GRAY K ; ELLIS V.** Activation of pro-BDNF by the pericellular serine protease plasmin [J. *Febs Letters,* 2008, vol. 582 (6), 907-910
- Cuprizone Model as a Tool for Preclinical Studies of the Efficacy of Multiple Sclerosis Diagnosis and Therapy[J. *Bulletin of Experimental Biology and Medicine,* 2015, vol. 159 (1), 111-115
- **KOMOLY S.** Experimental demyelination caused by primary oligodendrocyte dystrophy. Regional distribution of the lesions in the nervous system of mice [corrected].[J. *ideggyógyászati szemle,* 2005